# EUROPEAN PATENT APPLICATION

(11) **EP 3 733 267 A1**
(43) Date of publication of application: **04.11.2020**
(21) Application number: 20171946.5
(22) Date of filing: 28.04.2020
(51) Int. Cl.: B01D 61/14, B01D 65/02, C07K 1/34, C12N 15/10

(54) **DEVICE AND PROCESS FOR TREATMENT OF BIOLOGICAL MATERIAL**

(30) Priority: 29.04.2019 IT 201900006432
(71) Applicant: HYDRA S.r.l., 41037 Mirandola (MO) (IT)
(72) Inventor: BOSETTO, Antonio, 40137 BOLOGNA (IT); PIRAZZOLI, Paolo, 41030 SAN PROSPERO (MO) (IT)
(74) Representative: PGA S.p.A.

(57) **Abstract**

The present invention relates to a device (1) for the treatment of biological material (2) comprising a concentration circuit (3) having at least one concentration filter (20), at least one supply conduit (30) of the biological material (2), and a concentration section (40) interposed between separation filter (10) and concentration filter (20) and defining an inner volume configured to accommodate a fraction of interest (2a) of the biological material (2). A discharge conduit (50) is connected to the second access (22) of the concentration filter (20) downstream of the concentration section (40), and an infusion line (60) is connected to the concentration circuit (3). The concentrated fraction of interest (2a) of biological material (2) may be withdrawn from a collection channel (70) or by removing the entire concentration section (40) from the rest of the circuit.

## Description

### FIELD OF THE INVENTION

The present invention regards a device for the treatment of biological material, in particular for the concentration and the isolation of biological material of interest by means of filtering. The invention also comprises the process for treating the biological material. The biological material may be of any origin, plant, animal or human, previously separated from the organism of origin - plant, animal, or human body - before being subjected to the treatment pursuant to the invention.

### STATE OF THE ART

Known in the medical and pharmaceutical sector are devices and processes adapted to concentrate a particular type of cells or particles of interest, dispersed in a liquid phase by means of a two-stage size filtration. In particular, devices are known comprising a pair of membrane filters arranged consecutively one after the other, and each having a different filtering capacity: the material to be filtered consecutively crosses such filters, in a manner such that a first filter determines the filtration of the larger-size particles, while a second filter determines the filtration of particles having a reduced size, in this manner causing the concentration of particles of interest having an intermediate size.

The document WO2010130303A1 is aimed for a device and relative process for the separation of cells of biological nature from a liquid solution. The device provides for a structure comprising a first and a second filter that are separable from each other and having different filtering properties, a suction line and a delivery line. The device provides for a step for suctioning the solution to be filtered, in a manner so as to determine the passage thereof through the first filter and, subsequently, through the second filter: in particular, the first filter is configured to retain larger-size discard components, while the second filter is configured to retain the particles of interest. Once the step for isolating the particles of interest is completed, the first filter is separated from the second filter; a subsequent step provides for supplying a fluid coming from the delivery line towards the second filter, crossing the latter in an opposite direction with respect to the preceding step so as to be mixed with the particles of interest and allow the collection thereof in a suitable container.

A further United States document US4420398A discloses a filtration method for the extraction of antiviral substances from a liquid solution, by means of a double size filtration with membrane: the portion interposed between the two filters determines a zone of concentration of the particles of interest. The fluid portion crossing the second filter is collected in a discard tank. A fluid is introduced directly upstream into the tank containing the substance to be filtered. Upstream of the first and second filter, a respective recirculation is present that is adapted to determine a flow tangential to the membrane of the respective filter.

The devices and the processes of the state of the art have several problems and limits.

In particular, the system described in the document WO2010130303A1 provides for the complete opening of the structure of the device and the removal of the first filter so as to allow withdrawing the particles of interest, exposing the latter to contamination, unless the entire apparatus is housed and operates in a sterile chamber. In addition, the system described in the document WO2010130303A1 has extremely poor flexibility and is not very suitable to being automated, in fact resulting exclusively usable in laboratory environments.

The device pursuant to document US4420398A provides for the introduction of the dilution fluid directly within a main container housing the material to be filtered; since the material to be filtered is typically provided in sealed and sterile bags, any one tampering or opening can compromise the sterility and cause the contamination of the main container, in addition to requiring an additional connection operation. In addition, the quantity of dilution fluid insertable in the main container is inevitably tied with the volume of the main container housing the material to be filtered: in other words the dilution fluid can at most compensate for the volume of material that progressively leaves the main container. Finally, the described system does not ensure the control of the ratio between the material to be filtered and the dilution fluid, reducing both the operating flexibility of the system and highlighting problems tied with the possible obstruction of the filters during the filtration process. The method of the document US4420398A, even if providing for the possibility of creating a flow tangential to the membranes of the filters, does not completely resolve the abovementioned problem since the actual concentration of the entering material is hard to control, requiring a recurring forced removal and cleaning of the filters.

### CONVENTIONS

In the present description, the filtering capacity of a membrane is expressed in terms of porosity and for example determined through porometric techniques, which in statistical terms determine a size characteristic of the average pores of the membrane commonly measured in µm.

The cut-off thresholds referred to in this document in relation to the filtering properties of the membranes are expressed in terms of size porosity of the membrane itself.

It is observed that in the present detailed description, corresponding parts illustrated in the various figures are indicated with the same reference numbers. The figures could illustrate the object of the invention by means of representations that are not in scale; therefore, parts and components illustrated in the figures relative to the object of the invention might exclusively regard schematic representations.

The terms upstream and downstream make reference to an advancement direction of the fluid containing the material to be treated or of an auxiliary dilution fluid.

### DEFINITIONS

### Control unit

The device and the process for concentrating biological material described and claimed herein may respectively comprise and use a control unit set for controlling the various steps or operations implemented by the device 1 or executed by the treatment process described or claimed. There device may have (and the process may use) only one control unit or there may be a plurality of separate control units depending on design selections and operating requirements.

With the term control unit, it is intended a component of electronic type which may comprise at least one of the following: a digital processor (e.g. comprising at least one selected from the group from among: CPU, GPU, GPGPU), a memory (or memories), a circuit of analog type, or a combination of one or more digital processing units with one or more circuits of analog type. The control unit may be "configured" or "programmed" for executing several steps: in practice this may be attained with any means which allows configuring or programming the control unit. For example, in the case of a control unit comprising one or more CPUs and one or more memories, one or more programs may be stored in appropriate memory banks connected to the CPU or to the CPUs; the program or programs contain instructions which, when executed by the CPU or CPUs, program or configure the control unit or units to execute the operations described in relation to the control unit. Alternatively, if the control unit is or comprises circuitry of analog type, then the circuit of the control unit may be designed for including circuitry configured, in use, for processing electrical signals in a manner such to execute the steps relative to the control unit. The control unit may comprise one or more digital units, for example of microprocessor type, or one or more analog units, or a suitable combination of digital and analog units; the control unit may be configured for coordinating all the actions necessary for executing an instruction or sets of instructions.

### OBJECT OF THE INVENTION

Object of the present invention is to substantially solve at least one of the drawbacks and/or limitations of the preceding solutions.

A first object of the invention is to provide a closed system for the concentration of biological material by means of use of filters having different cut-off thresholds.

A further object of the invention is to arrange a system for the concentration of biological material free of risks of contamination both of the material to be filtered and of the obtained concentrated product.

A further object of the invention is to provide a system for the concentration of biological material capable of preventing the obstruction of the filters and of allowing the continuous or cyclic cleaning thereof, so as to ensure an efficient operation of the filtering membrane.

A further object of the invention is to provide a system for the concentration of biological material which allows a constant operation, prolonged over time, free of continuous and recurring interruptions.

A further object of the invention is to provide a system for the concentration of biological material which allows a quick and easy removal and substitution of the filter or filters.

A further object of the invention is to provide a system for the concentration of biological material which allows the installation of a variety of filters having of different cut-off thresholds.

A further object of the invention is to provide a system for the concentration of biological material which allows the installation of pairs of filters or of groups of filters having different cut-off ratios.

Finally, an auxiliary object of the invention is to provide a technical solution that allows isolating concentrated material in a volume portion, a container also usable as part of a bioreactor, e.g. for the subsequent cellular expansion, allowing the removal of the entire container, or bioreactor, where the concentration is actuated, preventing the extraction of the cellular concentrate in order to then insert it in a separate container or bioreactor.

These objects and still others, which will be clearer from the following description, are substantially attained by a circuit, a system and a process for the concentration of biological material in accordance with that expressed in one or more of the enclosed claims and/or of the following aspects, taken separately or in any one combination with each other or in combination with any one of the enclosed claims and/or in combination with any one of the further aspects or characteristics described hereinbelow.

### SUMMARY

Aspects of the invention are described hereinbelow.
A 1st aspect regards a device (1) for treatment of biological material (2) comprising:
   - a concentration circuit (3) having:
      at least one supply conduit (30) comprising a first end (31) connectable to a supply unit (5) of biological material (2);
      at least one concentration filter (20) in fluid communication with the supply conduit (30) and comprising a first and a second access (21, 22), and a filtering membrane (23) interposed between said first and second access (21, 22);
      a concentration section (40) located upstream of the at least one concentration filter (20), said concentration section (40) defining an inner volume configured to accommodate a fraction of interest of the biological material (2);
      at least one discharge conduit (50) comprising a first end (51) connectable to a discharge unit (6), and at least one second end (52) connected to the second access (22) of the at least one concentration filter (20) downstream of the concentration section (40),
   - at least one infusion line (60) comprising a first end (61) connectable to an auxiliary fluid source (8) and at least one second end (62) connected to the concentration circuit (3).
In a 2nd aspect according to the preceding aspect the concentration circuit (3) also has at least one separation filter (10) comprising a first and a second access (11, 12), and a filtering membrane (13) interposed between said first and second access (11, 12).
In a 3rd aspect according to the preceding aspect a second end (32) of the at least one supply conduit (30) is connected to the first access (11) of the at least one separation filter (10); and the at least one concentration filter (20) is located downstream of the separation filter (10).
In a 4th aspect according to the 2nd or 3rd aspect, concentration filter(s) (20; 20a, 20b) has a lower cut-off threshold with respect to a cut-off threshold of the filtering membrane (13) of the separation filter (10).
In a 5th aspect according to the 2nd or 3rd or 4th aspect, said concentration section (40) is interposed between the at least one separation filter (10) and the at least one concentration filter (20) and is connected between the second access (12) of the at least one separation filter (10) and the first access (21) of the at least one concentration filter (20).
In a 6th aspect according to any one of the preceding aspects from the 2nd to the 5th, the filtering membrane (13) of the separation filter (10) has a cut-off threshold comprised between 50 and 200 µm, and/or wherein the filtering membrane (23; 23a, 23b) of the concentration filter(s) (20; 20a, 20b) has a cut-off threshold comprised between 3 and 10 µm.
In a 7th aspect according to any one of the preceding aspects, the concentration section (40) defines a concentration volume, optionally comprised between 10 and 100 cm3, communicating with the rest of the concentration circuit (3) exclusively through the membranes (13, 23) respectively of the at least one separation filter (10) and of the at least one concentration filter (20).
In an 8th aspect according to any one of the preceding aspects the concentration circuit (3) is made of a single plastic component of the sterile or sterilizable disposable type.
In a 9th aspect according to any one of the preceding aspects, the infusion line (60) has the at least one second end (62) connected to at least one from among:
   the supply conduit (30) at a connection node, the latter located upstream of the first access of the separation filter (10) to allow, at least in an operative condition, the mixing of the biological material (2) with the auxiliary fluid (8) upstream of the separation filter (10);
   a connection port (68) disposed tangentially and entering a first chamber (10') of the separation filter (10) which also comprises a second chamber (10") separated from the first chamber (10') by means of the filtering membrane (13) of the separation filter (10), said connection port (68) being configured to define, at least in an operative condition, a tangential flow with respect to the surface of the filtering membrane (13);
   the concentration section (40) to allow, at least in an operative condition, an injection of auxiliary fluid (8) directly within the concentration section (40);
   the second access (22) of the at least one concentration filter (20) to allow, at least in an operative condition, the flow of the auxiliary fluid (8) through said concentration filter (20) inwardly from the concentration section (40).
In a 10th aspect according to any one of the preceding aspects, the device comprises a withdrawal conduit (70) having a first end (71) connectable to a withdrawal unit (73), and a second end (72) connected to the concentration section (40).
In an 11th aspect according to the preceding aspect said withdrawal conduit (70) is configured to allow withdrawing the fraction of interest of the biological material (2) from the concentration section (40).
In a 12th aspect according to the 10th or 11th aspect, the concentration section is removable from the rest of the circuit (3).
In a 13th aspect according to the preceding aspect, the concentration section comprises connectors (40b) removably engageable with corresponding counter-connectors carried by the circuit (3), optionally by the concentration filter(s) (20; 20a, 20b) and by the separation filter (10).
In a 14th aspect according to the preceding aspect each of the connectors (40b) incorporates a check valve which closes the respective connector (40b) as soon as it is disengaged from the circuit (3) or in which valves (40c) are active on the concentration section (40) and each operating adjacent to the respective connector (40b).
In a 15th aspect according to any one of the preceding aspects, the concentration section (40) is made in the shape of an elongated hollow body and comprises a rectilinear axis tube (40a), optionally corrugated or helically shaped, extended between the concentration filter (20) and the selection filter (10).
In a 16th aspect according to any one of the preceding aspects, the concentration filter (20) comprises a first and a second concentration filter (20a, 20b), in which each first and second concentration filter (20a, 20b) has a respective first access (21a, 21b) and a respective second access (22a, 22b).
In a 17th aspect according to the preceding aspect:
   a. the first access (21a, 21b) of each first and second concentration filter (20a, 20b) is connected, optionally directly connected, to the concentration section (40),
   b. the second access (22a, 22b) of each first and second concentration filter (20a, 20b) is connected, optionally directly connected, to the at least one infusion line (60),
   c. the second access (22a, 22b) of each first and second concentration filter (20a, 20b) is connected, optionally directly connected, to the discharge conduit (50).
In an 18th aspect according to any one of the preceding aspects from the 9th to the 17th, the at least one infusion line (60) comprises:
   a common primary section (65) terminating in said first end (61) of the infusion line (60);
   a first secondary section (66a) which terminates in a second end (62) of the infusion line (60) and which connects said primary section (65) with the second access (22a) of the first concentration filter (20a);
   a second secondary section (66b) which terminates in another second end (62) of the infusion line (60) and which connects said primary section (65) with the second access (22b) of the second concentration filter (20b).
In a 19th aspect according to any one of the preceding aspects the discharge conduit (50) of the concentration circuit (3) comprises:
   a common primary section (55) terminating in said first end (51) of the discharge conduit (50);
   a first secondary section (56a) which terminates in a second end (52) of the discharge conduit (50) and which connects said primary section (55) with the second access (22a) of the first concentration filter (20a);
   a second secondary section (56b) which terminates in another second end (52) of the discharge conduit (50) and which connects said primary section (55) with the second access (22b) of the second concentration filter (20b).
In a 20th aspect according to any one of the preceding two aspects, each of said first and second secondary section (66a, 66b) of the infusion line (60) comprises at least one respective interception member (80a, 80b) which may be configured at least in an open position and in a closed position, respectively to allow and prevent, optionally to reduce, the flow of the auxiliary fluid (8) through said first and second secondary section (66a, 66b) of the infusion line (60). In particular the at least one respective interception member (80a, 80b) is part of infusion flow regulator, which is preferably controlled by a control unit of the device (1).
In a 21st aspect in accordance with any one of the preceding three aspects, each first and second secondary section (56a, 56b) of the discharge conduit (50) comprises at least one respective interception member (81a, 81b) which may be configured at least in an open position and in a closed position, respectively to allow and prevent, optionally to reduce, the flow of the waste fluid (7) through said first and second secondary section (56a, 56b) of the discharge conduit (50).
   In particular the at least one respective interception member (81a, 81b) is part of a discharge flow regulator, which is preferably controlled by a control unit of the device (1).
In a 22nd aspect according to any one of the preceding aspects the concentration section (40) comprises a tubular main body (40a) having symmetrical conformation.
In a 23rd aspect according to the preceding aspect the concentration section comprises the main body, optionally U-shaped or V-shaped, having two symmetrically opposed sections (40e) emerging from a base section (40d); and in which each of the opposite sections (40e) is connected with a respective of said first and second concentration filter (20a, 20b).
In a 24th aspect according to the preceding aspect the base section (40d), preferably at a central position thereof, is connected with the withdrawal conduit (70).
In a 25th aspect according to the aspect 23rd or 24th, the base section (40d), preferably at a central position thereof, is connected with delivery channel (90) and/or with the selection filter (10).
In a 26th aspect according to any one of the preceding aspects in which the discharge conduit (50) comprises at least one respective flow interception member (81; 81a, 81b) which may be configured at least in an open position and in a closed position, respectively to allow or prevent, optionally to reduce, the flow or flow of a fraction to be discarded of the biological material (2) through said discharge conduit (50).
   In particular the at least one respective interception member (81; 81a, 81b) is part of a discharge flow regulator, which is preferably controlled by a control unit of the device (1).
In a 27th aspect according to any one of the preceding aspects, the/a withdrawal conduit (70) is connected with the concentration circuit (3) for collecting the fraction of interest of the biological material and comprises at least a respective interception member (82) which may be configured at least in an open position and in a closed position, respectively to allow or prevent, optionally to reduce, the flow of the fraction of interest (2a) of the biological material (2) through said withdrawal conduit (70).
   In particular the at least one respective interception member (82) is part of withdrawal flow regulator, which is preferably controlled by a control unit of the device (1).
In a 28th aspect according to any one of the preceding aspects the device provides for means for adjusting the flow or infusion flow regulator (80; 100; 100, 80a, 80b), optionally comprising at least one pump and one or more valves, configured to be operatively active on the infusion line.
In a 29th aspect according to any one of the preceding aspects the device comprises a control unit (110) connected with flow adjustment means or infusion flow regulator (80) active on the infusion line and with a discharge flow regulator, for example with at least one interception member (81), active on the discharge conduit (50), the control unit (110) being configured to determine the emptying of the concentration section (40) by controlling the flow adjustment means or infusion flow regulator active on the infusion line in order to inject into the concentration section (40) a predetermined volumetric quantity of auxiliary fluid (8), and by controlling said discharge flow regulator, in particular the at least one interception member (81), active on the discharge conduit (50) to determine the exit from the discharge conduit (50) of an equivalent volumetric quantity of biological material to be discarded in a manner such that the material entering the concentration section proceeds in an orderly manner from the inlet to the outlet of the concentration section itself.
In a 30th aspect according to any one of the aspects from the 16th to the 29th, the device comprises a control unit (110) connected with flow adjustment means or infusion flow regulator (100a; 100a, 80a, 80b) active on the infusion line and with a discharge fluid flow regulator, in particular with at least one interception member (81a, 81b), active on the discharge conduit (50), the control unit (110) being configured to arrange the circuit (3) alternatively in:
   a. a first operative condition in which the control unit controls the flow adjustment means or infusion flow regulator (100a; 100a, 80a, 80b) active on the infusion line in order to inject into the concentration section (40) a predetermined volumetric quantity of auxiliary fluid (8) through the first concentration filter (20a), and control said discharge flow regulator, in particular said at least one interception member (81a, 81b), active on the discharge conduit (50) to determine the exit from the discharge conduit (50) of an equivalent volumetric quantity of biological material to be discarded through the second concentration filter (20b);
   b. a second operative condition in which the control unit controls the flow adjustment means or infusion flow regulator (100a; 100a, 80a, 80b) active on the infusion line in order to inject into the concentration section (40) a predetermined volumetric quantity of auxiliary fluid (8) through the second concentration filter (20b), and control said discharge flow regulator, in particular said at least one interception member (81a, 81b) active on the discharge conduit (50) to determine the exit from the discharge conduit (50) of an equivalent volumetric quantity of biological material to be discarded through the first concentration filter (20a).
In a 31st aspect according to any one of the preceding aspects the device comprises a delivery channel (90) which connects the second access (12) of the at least one separation filter (10) to an intake port (41) of the concentration section (40).
In a 32nd aspect according to the preceding aspect each of the first accesses (21a, 21b) of the first and second concentration filters (20a, 20b) is connected respectively to a first and to a second connection port (42a, 42b) of the concentration section (40), said intake port (41) being optionally equidistant from said first and second connection port (42a, 42b).
In a 33rd aspect according to any one of the preceding aspects the device includes the supply unit, which is defined by a first tank (5) connected to the first end (31) of the supply conduit (30).
In a 34th aspect according to any one of the preceding aspects the device includes the auxiliary fluid source, which is defined by a second tank (9) connected to the first end (61) of the infusion line (60).
In a 35th aspect according to any one of the preceding aspects the device includes the discharge unit, which defined by a collection tank (6) connected to the first end (51) of the discharge conduit (50).
In a 36th aspect according to any one of the preceding aspects the device includes the withdrawal unit, which is defined by a concentration tank (73) connected to the first end (71) of the withdrawal conduit (70).
In a 37th aspect according to any one of the 4 preceding aspects, the device includes the concentration circuit (3), the infusion line (60), the first tank, the second tank, the collection tank and the optional withdrawal tank connected to each other in a fluid-tight manner.
In a 38th aspect according to any one of the preceding aspects the device is configured to communicate with the external environment of the device itself exclusively through one or more gas vent lines (83).
In a 39th aspect according to the preceding aspect the one or more vent lines are arranged at one of opposite chambers (namely the chambers defined in the separation filter at opposite sides of the membrane) of the at least one separation filter (10) and/or at at least one of opposite chambers (namely the chambers defined at opposite sides of the membrane of each concentration filter) of the at least one concentration filter (20).
In a 40th aspect according to any one of the preceding 2 aspects, each vent line being provided with:
   - a respective hydrophobic filter configured to allow the passage of gas at the same time preventing passage of solids or liquids through the vent line, and/or
   - a respective ventilation valve which may be configured between an open position, in which it allows the discharge through the vent line of gas present within the device, and a closed position, in which it prevents the passage of gas through the vent line.
In a 41st aspect in accordance with any one of the preceding aspects the concentration circuit (3), the infusion line (60), the first tank, the second tank, the collection tank and the optional withdrawal tank define a single circuit in plastic material of the sterile or sterilizable disposable type.
In a 42nd aspect in accordance with any one of the preceding aspects dal 18th to the 41st the device is which may be configured in:
   - a/the first filtering condition in which:
      the interception member (80a) of the first secondary section (66a) of the infusion line (60) is arranged in the open position;
      the interception member (81a) of the first secondary section (56a) of the discharge conduit (50) is arranged in the closed position;
      the interception member (80b) of the second secondary section (66b) of the infusion line (60) is arranged in the closed position;
      the interception member (81b) of the second secondary section (56b) of the discharge conduit (50) is arranged in the open position.
In a 43rd aspect in accordance with the preceding aspect, the device is which may be configured in:
   - a/the second filtering condition, alternative to the first filtering condition, in which:
      the interception member (80a) of the first secondary section (66a) of the infusion line (60) is arranged in the closed position;
      the interception member (81a) of the first secondary section (56a) of the discharge conduit (50) is arranged in the open position;
      the interception member (80b) of the second secondary section (66b) of the infusion line (60) is arranged in the open position;
      the interception member (81b) of the second secondary section (56b) of the discharge conduit (50) is arranged in the closed position.
In a 44th aspect according to any one of the preceding 2 aspects, the device (1) is iteratively configurable in said first and second filtering conditions, in particular upon action of the control unit.
In a 45th aspect according to any one of the preceding aspects, in which the supply conduit (30) or the delivery channel (90) comprises an interception member (84) which may be configured at least in an open position and in a closed position, respectively to allow or prevent, optionally to reduce, the flow of fluid towards the concentration section (40).
In a 46th aspect according to the preceding aspect the treatment device (1) is configurable in an condition of withdrawal in which:
   the interception member (84) on the conduit (30) or on the delivery channel (90) is in closed position;
   at least one from between the interception member (80a, 80b) of the first and second secondary sections (66a, 66b) of the infusion line (60) is in open position;
   the interception member (81a, 81b) of the first and second secondary sections (56a, 56b) of the discharge conduit (50) is in closed position;
   the interception member (82) on the withdrawal conduit (70) is in open position.
In a 47th aspect according to any one of the preceding aspects, in which the concentration circuit (3) comprises at least one pump (100), optionally at least one peristaltic pump, active on at least one from among the supply conduit (30), the delivery channel (90) and the infusion line (60).
In a 48th aspect according to the preceding aspect, the at least one pump (100) comprises:
   a first pump (100a), optionally a first peristaltic pump, active on the primary section (65) of the infusion line (60) and configured to determine the flow of the auxiliary fluid (8) towards and through the at least one concentration filter (20), optionally through the first and the second concentration filter (20a, 20b); and
   a second pump (100b), optionally a second peristaltic pump, connected to the supply conduit (30) or to the delivery conduit (90) and configured to determine the flow of the biological material (2), optionally mixed with the auxiliary fluid (8), towards and through the at least one separation filter (10).
In a 49th aspect according to any one of the preceding aspects, in combination with the 18th aspect, a/the control unit (110) is connected at least:
   to each interception member (80a, 80b) of the first and second secondary section (66a, 66b) of the infusion line (60); and
   to each interception member (81a, 81b) of the first and second secondary section (56a, 56b) of the discharge conduit (50);
   said control unit (110) being configured to control, optionally to independently control, each of said interception members (81a, 81b, 80a, 80b) to be arranged in a respective open or closed position.
In a 50th aspect according to one of the preceding two aspects the control unit (110) is also connected to the first and to the second pump (100a, 100b) and configured to selectively control the activation thereof,
   and in which the control unit (110) is configured to perform a first procedure, defining the/a first filtering condition, comprising:
   activating the second pump (100b) to move biological material (2) towards the concentration section (40) through the at least one separation filter (10);
   controlling to the open position the interception member (80a) of the first secondary section (66a) of the infusion line (60);
   controlling to the closed position the interception member (81a) of the first secondary section (56a) of the discharge conduit (50);
   controlling to the closed position the interception member (80b) of the second secondary section (66b) of the infusion line (60);
   controlling to the open position the interception member (81b) of the second secondary section (56b) of the discharge conduit (50);
   activating the first pump (100a) to move auxiliary fluid (8) towards the concentration section (40) through the first concentration filter (20a).
In a 51st aspect according to one of the preceding three aspects the control unit (110) is also connected to the first and to the second pump (100a, 100b) and configured to selectively control the activation thereof, in which the control unit is configured to perform a second procedure, defining the/a second filtering condition, comprising:
   activating the second pump (100b) to move biological material (2) towards the concentration section (40) through the at least one separation filter (10);
   controlling to the closed position the interception member (80a) of the first secondary section (66a) of the infusion line (60);
   controlling to the open position the interception member (81a) of the first secondary section (56a) of the discharge conduit (50);
   controlling to the open position the interception member (80b) of the second secondary section (66b) of the infusion line (60);
   controlling to the closed position the interception member (81b) of the second secondary section (56b) of the discharge conduit (50);
   activating the first pump (100a) to move auxiliary fluid (8) towards the concentration section (40) through the second concentration filter (20b).
In a 52nd aspect according to any one of the preceding aspects from the 30th to the 51st, the/a control unit is configured to cyclically and alternately switch the device (1) from the first to the second filtering condition by controlling the passage from the first to the second operative condition or at predetermined time intervals or following the attainment of specific transmembrane pressure conditions at the first and/or second concentration filter.
In a 53rd aspect according to any one of the preceding aspects from the 30th to the 51st, the/a control unit is configured to cyclically and alternately switch the device (1) from the first to the second operative condition by controlling the passage from the first to the second operative condition following the attainment of specific transmembrane pressure conditions at the first and/or second concentration filter.
In a 54th aspect according to the preceding aspect the device comprises first pressure sensors (p3 and p4) respectively located upstream and downstream of the first concentration filter (20a) and second pressure sensors (p5 and p6) respectively located upstream and downstream of the second concentration filter (20b), the control unit (110) being configured for:
   a. controlling the device (1) in the first operative condition,
   b. receiving pressure signals from the first pressure sensors (p3, p4),
   c. then determining the transmembrane pressure TMP across the membrane of the first concentration filter,
   d. when the transmembrane pressure across the membrane of the first concentration filter exceeds a predetermined threshold indicative of an excessive clogging, controlling the passage to the second operative condition,
   e. controlling the device (1) in the second operative condition,
   f. receiving the pressure signals from the sensors (p5, p6),
   g. then determining the transmembrane pressure TMP across the membrane of the second concentration filter, h. when the transmembrane pressure across the second concentration filter exceeds a predetermined threshold indicative of an excessive clogging, controlling the passage to the first operative condition.
In a 55th aspect according to any one of the preceding aspects said/a control unit (110) is connected to the interception member (82) of the withdrawal conduit (70) and configured to control said interception member (82) to the open or closed position.
In a 56th aspect according to any one of the preceding aspects said/a control unit is connected to the interception member (84) of the supply conduit (30) or of the delivery channel (90) and configured to control said interception member (84) to the open or closed position.
In a 57th aspect according to the two preceding aspects, in the operative condition of withdrawal, the control unit (110) is configured per:
   stopping the second pump (100b);
   controlling to the closed position the interception member (84) of the supply conduit (30) or of the delivery channel (90);
   controlling to the open position at least one from between the interception member (80a, 80b) of the first and second secondary sections (66a, 66b) of the infusion line (60);
   controlling to the closed position the interception member (81a, 81b) of the first and second secondary sections (56a, 56b) of the discharge conduit (50);
   activating the first pump (100a) to determine the flow of the auxiliary fluid (8) entering the concentration section (40);
   controlling to the open position the interception member (82) of the withdrawal conduit (70).
A 58th aspect regards the use of a device according to any one of the preceding aspects in which the concentration section (40) may be removed.
A 59th aspect regards the use of a device according to any one of the preceding aspects in which the concentration section (40) may be used as part of a bioreactor for subsequent cellular expansion.
A 60th aspect regards a process for the treatment of biological material (2) using the treatment device according to any one of the preceding aspects.
In a 61st aspect in accordance with the preceding aspect said process comprises at least the following steps:
   carrying out a procedure for washing and removing air from the circuit (3);
   feeding the concentration section (40) biological material (2) which has undergone at least one first filtration stage;
   determining at least one second stage for filtering biological material (2), said second filtering stage taking place through the at least one concentration filter (20), to determine the concentration of a fraction of interest (2a) of the biological material (2) at the concentration section (40);
   infusing the auxiliary fluid (8) in the concentration circuit (3) through the infusion line (60) to determine a dilution of the biological material (2) with the auxiliary fluid (8) before the second filtering stage.
In a 62nd aspect according to the preceding aspect the process provides that the first filtration stage of the biological material (2) takes place through at least one separation filter (10) constituting part of the circuit (3) and located upstream of the concentration filter (20).
In a 63rd aspect according to any one of the three preceding aspects, the process uses the device pursuant to aspect 16, in which it is provided to infuse auxiliary fluid (8) in the concentration section (40), alternatively through the first or the second concentration filter (20a, 20b), and in which it is also provided to move the biological material (2) alternatively through the first or the second concentration filter (20a, 20b) in a manner such that when one of the first or second concentration filter (20a, 20b) is crossed by auxiliary fluid (8) entering the concentration section (40), the other from between the first and second filter (20a, 20b) being crossed by an undesired fraction of the biological material exiting from the concentration section (40).
In a 64th aspect according to any one of the preceding 4 aspects, the process comprises a step for withdrawing the fraction of interest (2a) of the biological material (2) from the concentration section (40).
In a 65th aspect according to the preceding aspect, said withdrawal step comprising:
   in a first alternative at least one sub-step of controlling to the open position an interception member (82) on the withdrawal conduit (70) to allow the flow of the fraction of interest (2a) of the biological material (2) along the withdrawal conduit (70) towards a withdrawal unit (73);
   or in a second alternative the separation of the concentration section from the rest of the circuit (3).
In a 66th aspect using the first alternative of the preceding aspect the interception members (80a, 80b) of the first and second secondary sections (66a, 66b) of the infusion line (60) are opened one at a time, in two separate steps, controlling the volume introduced in each of the two portions of the concentration section (40) so as to accurately measure the volumetric quantity of material pushed towards the withdrawal conduit (70).
In a 67th aspect according to any one of the preceding 7 aspects, after having concentrated the fraction of interest (2a) of the biological material (2) at the concentration section (40), the concentration section (40) is used as part of a bioreactor for subsequent cellular expansion of the cells of interest.
In a 68th aspect according to any one of the aspects from the 1st to the 59th, a/the control unit (110) is configured to perform the process pursuant to any one of the preceding 8 aspects.
A 69^{th} aspect concerns a device (1) for treatment of biological material (2) comprising:
   - a concentration circuit (3) having:
      at least one supply conduit (30) comprising a first end (31) connectable to a supply unit (5) of biological material (2);
      at least one concentration filter (20, 20a, 20b) in fluid communication with the supply conduit (30);
      a concentration section (40) in fluid communication with the supply conduit (30) upstream of the at least one concentration filter (20, 20a, 20b), said concentration section (40) being also connected to at least one first access of the concentration filter (20, 20a, 20b) and defining an inner volume configured to accommodate a fraction of interest of the biological material (2);
      at least one discharge conduit (50) comprising a first end (51) connectable to a discharge unit (6), and at least one second end (52) connected to at least one second access of the at least one concentration filter (20, 20a, 20b);
   - at least one infusion line (60) comprising a first end (61) connectable to an auxiliary fluid source (8) and at least one second end (62) connected to the concentration circuit (3).
In a 70^{th} aspect according to the 69^{th} aspect said concentration filter (20) comprises at least a first concentration filter (20a) and a second concentration filter (20b).
In a 71^{st} aspect according to any one of the preceding two aspects the first concentration filter (20a) has:
   at least one respective first access (21a) connected to the concentration section (40),
   at least one respective second access (22a),
   a respective filtering membrane (23) interposed between the first access (21a) and the second access (22a)
   of the first concentration filter (20a);
   wherein the at least one infusion line (60) is configured to be placed in fluid communication with at least one second access (22a) of the first concentration filter (20a) such that auxiliary fluid directed by the at least one infusion line into the first concentration filter (20a) via the at least one second access (22a) crosses the filtering membrane (23) of the first concentration filter (20a) to reach the concentration section (40);
   wherein the at least one discharge conduit (50) is configured to be placed into fluid communication with at least one second access (22a) of the first concentration filter (20a) for receiving an undesired fraction of the biological material leaving the concentration section (40) and crossing the filtering membrane (23) of the first concentration filter (20a).
In a 72^{nd} aspect according to any one of the preceding three aspects the second concentration filter (20b) has:
   at least one respective first access (21b) connected to the concentration section (40),
   at least one respective second access (22b),
   a respective filtering membrane (23) interposed between the first access (21b) and the second access (22b)
   of the second concentration filter (20b);
   wherein the at least one infusion line (60) is configured to be placed in fluid communication with at least one second access (22b) of the second concentration filter (20b) such that auxiliary fluid directed into the second concentration filter (20b) via the at least one second access (22b) crosses the filtering membrane (23) of the second concentration filter (20b) to reach the concentration section (40),
   wherein the at least one discharge conduit (50) is configured to be placed into fluid communication with at least one second access (22a) of the second concentration filter (20b) for receiving an undesired fraction of the biological material leaving the concentration section (40) and crossing the filtering membrane of the second concentration filter (20b).
In a 73^{rd} aspect according to any one of the preceding four aspects the concentration circuit (3) also comprises at least one separation filter (10) comprising a respective first and a respective second access (11, 12), and a filtering membrane (13) interposed between said first and second access (11, 12); wherein a second end (32) of the at least one supply conduit (30) is connected to the first access (11) of the at least one separation filter (10) and said concentration section (40) is interposed between the at least one separation filter (10) and the first and second concentration filters (20a, 20b).
In a 74^{th} aspect according to the preceding aspect the filtering membrane (23) of the first and second concentration filters (20a, 20b) has a cut-off threshold different from a cut-off threshold of the filtering membrane (13) of the separation filter (10).
In a 75^{th} aspect according to any one of the preceding two aspects the concentration section (40) is on one side connected or connectable to the second access (12) of the at least one separation filter (10) and on the other side connected or connectable to the first access (21a, 21b) of the first and second concentration filters (20a, 20b).
In a 76^{th} aspect according to any one of the preceding three aspects the filtering membrane (23) of the first and second concentration filters (20a, 20b) has lower cut-off threshold with respect to the cut-off threshold of the filtering membrane (13) of the separation filter (10).
In a 77^{th} aspect according to any one of the preceding four aspects the filtering membrane (13) of the separation filter (10) has cut-off threshold of between 50 and 200 µm, and the filtering membrane (23; 23a, 23b) of the first and second concentration filters (20; 20a, 20b) has cut-off threshold of between 3 and 10 µm.
In a 78^{th} aspect according to any one of aspects from the 69^{th} to the preceding aspect, the device comprises:
   at least one infusion flow regulator (80a, 80b) operative on the at least one infusion line (60) and configured to control flow of auxiliary fluid through the same at least one infusion line,
   at least one discharge flow regulator (81a, 81b) operative on the discharge conduit (50) and configured to control flow of undesired fraction of biological material through the same discharge conduit.
In a 79^{th} aspect according to the preceding aspect the device comprises:
   a control unit (110) connected to the infusion flow regulator (80a, 80b) and to the discharge flow regulator (81a, 81b) and configured to execute the following concentration cycle:
   control the infusion flow regulator (80a, 80b) to infuse auxiliary fluid (8) in the concentration section (40) alternatively through the first or second concentration filter (20a, 20b), and
   control the discharge flow regulator (81a, 81b) to move undesired fractions of biological material (2) out of the concentration section (40) alternatively through the first or second concentration filter (20a, 20b),
   the control unit (110) being configured to control the infusion flow regulator (80a, 80b) and the discharge flow regulator (81a, 81b) so that when one of the first and the second concentration filter (20a, 20b) is crossed by the auxiliary fluid (8) entering the concentration section (40), the other between the first and second filter (20a, 20b) is crossed by an undesired fraction of the biological material leaving the concentration section (40).
In a 80^{th} aspect according to the preceding aspect the control unit is configured for repeating the above cycle a plurality of times.
In a 81^{st} aspect according to any one of aspects from the 69^{th} to the preceding aspect the at least one infusion line (60) comprises:
   a common primary section (65) terminating in said first end (61) of the infusion line (60);
   a first secondary section (66a) which ends at a second end (62) of the infusion line (60) and which connects said primary section (65) with the second access (22a) of the first concentration filter (20a);
   a second secondary section (66b) which ends in another second end (62) of the infusion line (60) and which connects said primary section (65) with the second access (22b) of the second concentration filter (20b);
   and wherein the discharge conduit (50) of the concentration circuit (3) comprises:
   a common primary section (55) terminating in said first end (51) of the discharge conduit (50);
   a first secondary section (56a) which ends at a second end (52) of the discharge conduit (50) and which connects said primary section (55) with the second access (22a) of the first concentration filter (20a);
   a second secondary section (56b) which ends in another second end (52) of the discharge conduit (50) and which connects said primary section (55) with the second access (22b) of the second concentration filter (20b).
In an 82^{nd} aspect according to the preceding two aspects each of said first and second secondary sections (66a, 66b) of the infusion line (60) comprises at least one respective interception member (80a, 80b) which is part of the infusion flow regulator and which is configurable at least in an open position and in a closed position, respectively to allow or prevent flow of the auxiliary fluid (8) through the first and second secondary sections (66a, 66b) of the infusion line (60) respectively into the first and second concentration filter; and/or
   wherein each first and second secondary section (56a, 56b) of the discharge conduit (50) comprises at least one respective interception member (81a, 81b) which is part of the discharge flow regulator and which is configurable at least in an open position and in a closed position, respectively to allow and prevent flow of the waste fluid (7) out of the first and second concentration filters (20a, 20b) through said first and second secondary section (56a, 56b) of the discharge conduit (50).
In an 83^{rd} aspect according to any one of aspects from the 73^{rd} to the preceding aspect the inner volume of the concentration section (40) is of between 10 and 100 cm³ and communicates with the rest of the concentration circuit (3) exclusively through the membranes (13, 23) of the at least one separation filer (10) and of the first and second concentration filters (20a, 20b).
In an 84^{th} aspect according to any one of aspects from the 69^{th} to the preceding aspect the concentration circuit is a single plastic disposable component, optionally a single plastic sterile disposable component.
In an 85^{th} aspect according to any one of aspects from the 69^{th} to the preceding aspect the at least one infusion line (60) is also configured to be selectively placed in fluid communication with a first chamber (10') of the separation filter (10) to define, at least in one operative condition, a tangential flow with respect to the surface of the filtering membrane (13), the separation filter further comprising a second chamber (10") separated from the first chamber (10') by the filtering membrane (13) of the same separation filter (10).
In an 86^{th} aspect according to the preceding aspect the infusion line (60) has a section (66e) having a second end (62) connected to the first chamber (10') of the separation filter (10) and wherein an additional discharge line (66f) is also connected to the first chamber (10') of the separation filter, the or a control unit (110) being active on a wash flow regulator, optionally including a washing pump (88), active on the section (66e) for causing a tangential wash through the first chamber (10') of the separation filter and out of the additional discharge line (66f).
In an 87^{th} aspect according to the preceding aspect wherein the control unit is configured for unit operating the wash flow regulator either continuously or at regular intervals or at intervals determined by detection of a triggering event which may be either a user command received by the control unit or expiration of set timeout period or a detection of reaching a specific transmembrane pressure across the separation filter (10) as detected based on pressure signals from sensors (p1 and p2) positioned upstream and downstream the separation filter and communicatively connected to the control unit (110).
In an 88^{th} aspect according to any one of aspects from the 69^{th} to the preceding aspect, the device comprises a withdrawal conduit (70) having a first end (71) connectable to a withdrawal unit (73), and a second end (72) connected to the concentration section (40), said withdrawal conduit (70) being configured to allow withdrawing the fraction of interest of the biological material (2) from the concentration section (40).
In an 89^{th} aspect according to any one of aspects from the 69^{th} to the preceding aspect the concentration section is removable from the rest of the circuit (3) and comprises connectors (40b) removably engageable to corresponding counter-connectors carried by the first and second concentration filters (20a, 20b) and by the separation filter (10), and wherein each of the connectors (40b) incorporates a check valve which closes the respective connector (40b) as soon as it is disengaged from the circuit (3) or wherein valves (40c) are active on the concentration section (40) and operating each adjacent to the respective connector (40b).
In an 90^{th} aspect according to any one of the preceding aspects the concentration section (40) comprises a tubular main body (40a) having a symmetrical conformation, optionally U-shaped or V-shaped, comprising two symmetrically opposed sections (40e) emerging from a base section (40d).
In a 91^{st} aspect according to the preceding aspect each of the opposite sections (40e) is connected to a respective one of said first and second concentration filters (20a, 20b), while the base section 40d, preferably located in a central position, is connected to the withdrawal conduit (70) and optionally with the selection filter (10).
In a 92^{nd} aspect according to any one of aspects from the 69^{th} to the preceding aspect the/a withdrawal conduit (70) is connected with the concentration circuit (3) and configured for collecting the fraction of interest of the biological material, and a withdrawal flow regulator (82) is operative on the withdrawal conduit and configured to control flow of the fraction of interest (2a) of the biological material (2) through said withdrawal conduit (70), optionally the withdrawal flow regulator comprising at least one respective interception member (82) which is configurable at least in an open position and in a closed position, respectively to allow or prevent flow of the fraction of interest (2a) of the biological material (2) through said withdrawal conduit (70).
In a 93^{rd} aspect according to the preceding aspect the control unit is connected to the withdrawal flow regulator (82) and is further configured to:
   first command execution of said concentration cycle a plurality of times,
   then stop execution of the concentration cycle and command the withdrawal flow regulator (2) to extract at least part of the fraction of interest (2a) of the biological material (2) from the concentration section (40) through said withdrawal conduit (70).
In a 94^{th} aspect according to the preceding aspect the control unit (110) during execution of said concentration cycle is configured to arrange the circuit (3) alternatively in:
   a first filtering condition in which the control unit controls the infusion flow regulator active on the infusion line to inject into the concentration section (40) a predetermined volumetric quantity of auxiliary fluid (8) through the first secondary filter (20a), and control said discharge flow regulator active on the discharge conduit (50) to determine the exit from the discharge conduit (50) of an equivalent volumetric quantity of biological material to be discarded through the second secondary filter (20b);
   a second filtering condition in which the control unit controls the infusion flow regulator active on the infusion line to inject into the concentration section (40) a predetermined volumetric quantity of auxiliary fluid (8) through the second secondary filter (20b), and control said discharge flow regulator active on the discharge conduit (50) to determine the exit from the discharge conduit (50) of an equivalent volumetric quantity of biological material to be discarded through the first secondary filter (20a).
In a 95^{th} aspect according to any one of aspects from the 69^{th} to the preceding aspect the/a delivery channel (90) connects the second access (12) of the at least one separation filter (10) to an intake port (41) of the concentration section (40),
   each of the first accesses (21a, 21b) of the first and second concentration filters (20a, 20b) being connected respectively to a first and a second connection port (42a, 42b) of the concentration section (40), said intake port (41) being optionally equidistant from said first and second connection ports (42a, 42b).
In a 96^{nd} aspect according to any one of aspects from the 69^{th} to the preceding aspect the device comprises:
   the supply unit, which is a first tank (5) connected to the first end (31) of the supply conduit (30);
   the auxiliary fluid source, which is a second tank (9) connected to the first end (61) of the infusion line (60);
   the discharge unit, which is a collection tank (6) connected to the first end (51) of the discharge conduit (50);
   and optionally the withdrawal unit, which is a concentration tank (73) connected to the first end (71) of the withdrawal conduit (70);
   in particular wherein the concentration circuit (3), the infusion line (60), the first tank, the second tank, the collection tank and the optional withdrawal tank are connected to each other in a fluid-tight manner.
In a 97^{nd} aspect according to any one of aspects from the 69^{th} to the preceding aspect the device, is configured to communicate with the external environment of the device exclusively through one or more gas vent lines (83), optionally arranged at separation filter (10) and/or at one of the first and second concentration filters (20a, 20b), each vent line being provided with:
   - a respective hydrophobic filter configured to allow the passage of gas at the same time preventing the passage of solids or liquids through the vent line, and/or
   - a respective ventilation valve which may be configured between an open position, in which it allows the discharge through the gas vent line inside the device, and a closed position, in which gas flow is prevented from passing through the vent line.
In a 98^{th} aspect according to the preceding aspect the concentration circuit (3), the infusion line (60), the first tank, the second tank, the collection tank and the optional withdrawal tank define a single circuit in plastic material of the sterile or sterilizable disposable type.
In a 99^{th} aspect according to any one of aspects from the 94^{th} to the 98^{th} the control unit is configured to:
   - command the device in the first filtering condition in which:
      the interception member (80a) of the first secondary section (66a) of the infusion line (60) is arranged in the open position;
      the interception member (81a) of the first secondary section (56a) of the discharge conduit (50) is arranged in the closed position;
      the interception member (80b) of the second secondary section (66b) of the infusion line (60) is arranged in the closed position;
      the interception member (81b) of the second secondary section (56b) of the discharge conduit (50) is arranged in the open position;
   - command the device in the second filtering condition, alternative to the first filtering condition, in which:
      the interception member (80a) of the first secondary section (66a) of the infusion line (60) is arranged in the closed position;
      the interception member (81a) of the first secondary section (56a) of the discharge conduit (50) is arranged in the open position;
      the interception member (80b) of the second secondary section (66b) of the infusion line (60) is arranged in the open position;
      the interception member (81b) of the second secondary section (56b) of the discharge conduit (50) is arranged in the closed position;
   wherein the device (1) is iteratively configurable in said first and second filtering conditions under the control of said control unit.
In a 100^{th} aspect according to any one of aspects from the 69^{th} to the preceding aspect the device has a supply flow regulator, in particular operative on the supply conduit (30) or the delivery channel (90), configured to control flow of biological material to the concentration section (40).
In a 101^{st} aspect according to the preceding aspect the supply flow regulator comprising an interception member (84) which may be configured at least in an open position and in a closed position respectively to allow or prevent flow of biological material flow to the concentration section (40).
In a 102^{nd} aspect according to any one of aspects from the 69^{th} to the preceding aspect the/a control unit (110) is configured to put the device in a withdrawal condition in which the control unit controls the/an infusion flow regulator to deliver auxiliary fluid into the concentration section (40) through one of said first and second filters (20a, 20b), controls the/a supply flow regulator to stop flow of biological material through the delivery channel or supply conduit, controls the/a withdrawal flow regulator to collect the concentrated fraction of interest through the withdrawal conduit (70).
In a 103^{rd} aspect according to the preceding aspect the control unit in the withdrawal condition configures the device such that:
   the interception member (84) on the conduit (30) or on the delivery channel (90) is in the closed position;
   at least one of the interception members (80a, 80b) of the first and second secondary sections (66a, 66b) of the infusion line (60) is in the open position;
   the interception member (81a, 81b) of the first and second secondary sections (56a, 56b) of the discharge conduit (50) is in the closed position;
   the interception member (82) on the withdrawal conduit (70) is in the open position.
In a 104^{th} aspect according to any one of aspects from the 69^{th} to the preceding aspect, wherein the concentration circuit (3) has:
   a first pump (100a) part of the infusion flow regulator, optionally a first peristaltic pump, active on the primary section (65) of the infusion line (60) and configured to determine flow of the auxiliary fluid (8) towards and through the first and second concentration filters (20a, 20b); and
   a second pump (100b) part of the supply flow regulator, optionally a second peristaltic pump, connected to the supply conduit (30) or to the delivery conduit (90) and configured to determine the flow of the biological material (2) towards and through the at least one separation filter (10).
In a 105^{th} aspect according to any one of aspects from the 69^{th} to the preceding aspect, wherein a/the control unit (110) is connected at least:
   to each interception member (80a, 80b) of the first and second secondary sections (66a, 66b) of the infusion line (60); and
   to each interception member (81a, 81b) of the first and second secondary sections (56a, 56b) of the discharge conduit (50),
In a 106^{th} aspect according to the preceding aspect said control unit (110) is configured to control, optionally to independently control, each of said interception members (81a, 81b, 80a, 80b) to be arranged in a respective open or closed position.
In a 107^{th} aspect according to any one of the preceding three aspects the control unit (110) is further connected to the first and the second pump (100a, 100b) and configured to selectively control the activation thereof,
   and wherein the control unit (110) is configured to perform a first procedure, defining the/a first filtering condition, comprising:
   activating the second pump (100b) to move biological material (2) towards the concentration section (40) through the at least one separation filter (10);
   controlling to the open position the interception member (80a) of the first secondary section (66a) of the infusion line (60);
   controlling to the closed position the interception member (81a) of the first secondary section (56a) of the discharge conduit (50);
   controlling to the closed position the interception member (80b) of the second secondary section (66b) of the infusion line (60);
   controlling to the open position the interception member (81b) of the second secondary section (56b) of the discharge conduit (50);
   activating the first pump (100a) to move auxiliary fluid (8) towards the concentration section (40) through the first concentration filter (20a); and/or
   and wherein the control unit is configured to perform a second procedure, defining the/a second filtering condition, comprising:
   activating the second pump (100b) to move biological material (2) towards the concentration section (40) through the at least one separation filter (10);
   controlling to the closed position the interception member (80a) of the first secondary section (66a) of the infusion line (60);
   controlling to the open position the interception member (81a) of the first secondary section (56a) of the discharge conduit (50);
   controlling to the open position the interception member (80b) of the second secondary section (66b) of the infusion line (60);
   controlling to the closed position the interception member (81b) of the second secondary section (56b) of the discharge conduit (50);
   activating the first pump (100a) to move auxiliary fluid (8) towards the concentration section (40) through the second concentration filter (20a).
In a 108^{th} aspect according to any one of aspects from the 94^{th} to the preceding aspect the control unit is configured to switch the device (1) from the first to the second filtering condition either at predetermined time intervals or upon detection of a transmembrane pressure threshold across the membrane of the second concentration filter, and to switch the device (1) from the second to the first filtering condition either at predetermined time intervals or upon detection of a transmembrane pressure threshold across the membrane of the first concentration filter.
In a 109^{th} aspect according to the preceding aspect first pressure sensors (p3 and p4) are provided, respectively located upstream and downstream of the first concentration filter (20a) and second pressure sensors (p5 and p6) respectively located upstream and downstream of the second concentration filter (20b), the control unit 110 being configured for cyclically repeating the following steps:
   a. controlling the device (1) in the first filtering condition,
   b. receiving pressure signals from the second pressure sensors (p5, p6),
   c. then determining the transmembrane pressure across the membrane of the second concentration filter,
   d. when the transmembrane pressure across the second concentration filter membrane exceeds a predetermined threshold indicative of an excessive clogging, commanding switch of the device to the second filtering condition,
   e. controlling the device (1) in the second filtering condition,
   f. receiving the pressure signals from the first pressure sensors (p3, p4),
   g. then determining the transmembrane pressure across the membrane of the first concentration filter,
   h. when the transmembrane pressure across the first concentration filter membrane exceeds a predetermined threshold indicative of an excessive clogging, commanding switch of the device to the first filtering condition.
In a 110^{th} aspect according to any one of the preceding six aspects said control unit (110) is connected to the interception member (82) of the withdrawal conduit (70) and configured to control said interception member (82) to the open or closed position,
   said control unit (110) being connected to the interception member (84) of the supply conduit (30) or of the delivery channel (90) and configured to control said interception member (84) to the open or closed position,
   wherein, in the withdrawal condition, the control unit (110) is configured for:
   stopping the second pump (100b);
   controlling to the closed position the interception member (84) of the supply conduit (30) or of the delivery channel (90);
   controlling to the open position at least one of the interception member (80a, 80b) of the first and second secondary sections (66a, 66b) of the infusion line (60);
   controlling to the closed position the interception member (81a, 81b) of the first and second secondary sections (56a, 56b) of the discharge conduit (50);
   activating the first pump (100a) to determine the flow of the auxiliary fluid (8) entering the concentration section (40);
   controlling to the open position the interception member (82) of the withdrawal conduit (70).
A 111^{th} aspect concerns a process of treatment of biological material (2) using the treatment device according to any one of aspects from the 64^{th} to the preceding aspect.
In a 112^{th} aspect according to the preceding aspect the process comprises at least the following steps:
   carrying out a priming procedure for washing and removing air from the circuit (3);
   feeding to the concentration section (40) biological material (2) which has undergone at least a first filtration stage;
   determining at least one second filtration stage of biological material (2), said second filtration stage taking place through the at least one concentration filter (20), to determine the concentration of a fraction of interest (2a) of the biological material (2) at the concentration section (40); optionally wherein the first filtration stage of the biological material (2) takes place through at least one separation filter (10) forming part of the circuit (3) and placed upstream of the concentration filter (20);
   infusing the auxiliary fluid (8) into the concentration section (40), alternatively through the first or second concentration filter (20a, 20b), wherein when one of the first or the second concentration filter (20a, 20b) is crossed by the auxiliary fluid (8) entering the concentration section (40), the other between the first and second filter (20a, 20b) is crossed by an undesired fraction of the biological material leaving the concentration section (40).
In a 113^{th} aspect according to the preceding aspect the process comprises a step for withdrawing a fraction of interest (2a) of the biological material (2) from the concentration section (40).
In a 114^{th} aspect according to the preceding aspect, said step of withdrawing the fraction of interest comprising: in a first alternative, at least one sub-step of causing the flow of the fraction of interest (2a) of the biological material (2) along the withdrawal conduit (70) towards a withdrawal unit (73); or, in a second alternative, separation of the concentration section (40) from the rest of the circuit (3).
In a 115^{th} aspect according to the preceding aspect, and in said first alternative, causing the flow of the fraction of interest along the withdrawal line includes the following sub-steps:
   preventing flow of fluid through the supply line,
   causing flow of auxiliary fluid through at least one of the first and second concentration filters to push the fraction of interest out of the concentration section through the withdrawal conduit.
In a 116^{th} according to the preceding aspect the process comprises controlling the volume of auxiliary fluid introduced into the concentration section (40) during said step of withdrawing the fraction of interest so as to accurately measure the volumetric quantity of material pushed towards the discharge conduit (50).

### BRIEF DESCRIPTION OF THE DRAWINGS

Several embodiments and several aspects of the invention will be described hereinbelow with reference to the enclosed drawings, provided only as a non-limiting example in which:
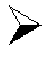 Figure 1 is a circuitry schematic view of a first embodiment of a device for treating biological material according to aspects of the invention;
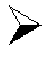 Figure 2 is a circuitry schematic view of a variant of the first embodiment of a device for treating biological material according to aspects of the invention;
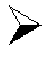 Figure 3 is a circuitry schematic view of a further variant of the first embodiment of a device for treating biological material according to aspects of the invention;
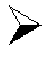 Figure 3A and figure 3B schematically show possible structures that may be conferred to the concentration section 40 when the latter is separable from the device pursuant to figures 1 to 3;
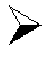 Figure 4 is a circuitry schematic view of a second embodiment of a device for treating biological material in a predetermined operative condition according to aspects of the invention;
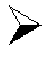 Figure 4A is a circuitry schematic view of a variant of the second embodiment of a device for treating biological material in a predetermined operative condition in which the concentration of the biological material being treated takes place, according to aspects of the invention;
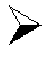 Figure 4B is a circuitry schematic view of the second embodiment of a device for treating biological material in a further operative condition in which the extraction of the concentrated biological material takes place, according to aspects of the invention;
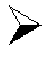 Figure 5A and figure 5B schematically show possible structures that may be conferred to the concentration section 40 when the latter is separable from the device pursuant to figures 4, 4A and 4B.

### DETAILED DESCRIPTION

### Device for the treatment of biological material

Reference number 1 indicates a device for the treatment of biological material 2 as shown in the enclosed figures: without prejudice to the general nature of its possible use, the device can for example be used for isolating, by means of a sequence of filtering operations, cells or cellular material of interest present within a fluid of biological material. The device has particular application for the concentration of cells coming from stromal mesenchyme, the latter massively present in the subcutaneous fat: in particular it is observed that in the residues of processing of the subcutaneous fat for lipofilling procedures, one finds, together with blood and oil residues of various type, a high concentration of isolated stromal mesenchymal cells, selectable by size, usable in regenerative medicine or advanced therapy procedures, as well as in research activities in the laboratory.

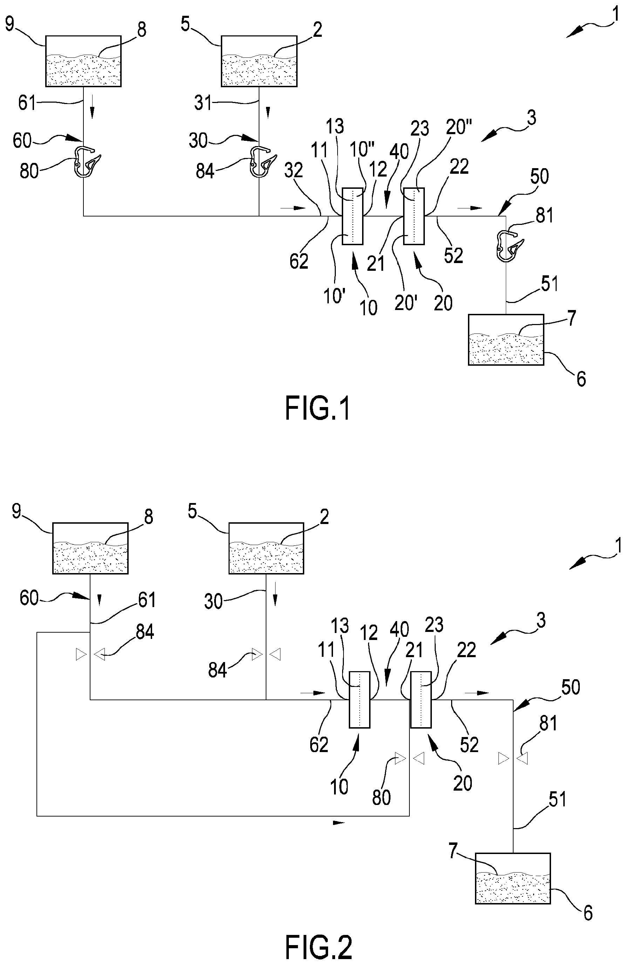

The device 1, shown in the enclosed figures 1 to 6, comprises a concentration circuit 3 preferably having a separation filter 10. For description ease, in the enclosed figures, examples are represented in which at least one separation filter 10 is always provided since this case represents the most complete integrated solution; nevertheless, the device 1 of the invention could lack separation filter 10, for example if the material 2 was previously treated in another site or if it intrinsically lacks elements of larger size than those that one had intended to concentrate. The separation filter 10 has a first and a second access 11, 12, divided by a filtering membrane 13 interposed between the two accesses. The separation filter 10 has an external casing made of plastic or metal material and defining at least part of the first and second accesses 11, 12; the external casing also defines an inner volume housing the filtering membrane 13: the latter divides the inner volume into a first chamber 10' interposed between the filtering membrane 13 and the first access 11, and a second chamber 10" interposed between the filtering membrane 13 and the second access 12. The separation filter may comprise at least one vent for the air present in the first and/or in the second chamber 10', 10" and provided with a respective hydrophobic membrane then capable of eliminating possible air or gas present in the circuit (e.g. during a non-further described priming procedure) without allowing any exit of liquid and maintaining the closure of the circuit with respect to possible contaminating particles present in the external environment. The filtering membrane 13 is interposed between the first and the second chamber 10', 10": during an operative condition, the biological material is then forced to move through the membrane 13 of the separation filter 10 in order to pass from the first to the second chamber 10', 10", defining a first stage of filtering the fluid or mass of incoming biological material 2.

The concentration circuit 3 comprises at least one concentration filter 20. In the illustrated solutions where a separation filter 10 is provided for, the concentration filter 20 is located downstream of the separation filter 10 (with respect to a direction of flow of the incoming biological material) and in turn has a first and a second access 21, 22: a filtering membrane 23 is interposed between the first and the second access 21, 22 of the concentration filter 20. The concentration filter 20 has an external casing made of plastic or metal material defining at least part of the first and second accesses 21, 22: the external casing defines an inner volume housing the filtering membrane 23; the latter divides the inner volume into a first chamber 20' interposed between the filtering membrane 23 and the first access 21, and a second chamber 20" interposed between the filtering membrane 23 and the second access 22. The filtering membrane 23 is then interposed between the first and the second chamber 20', 20": during an operative condition, the biological material is then forced to move through the membrane 23 of the concentration filter 20 in order to pass from the first to the second chamber 20', 20", defining a second stage of filtering the biological material. The concentration filter may comprise at least one vent for air present in the first and/or in the second chamber 20', 20" and provided with a respective hydrophobic membrane capable of eliminating possible air or gas present in the circuit (for example during a non-described priming procedure) without allowing any exit of liquid and maintaining the closure of the circuit with respect to possible contaminating particles present in the external environment.

The filtering membrane 23 of the concentration filter 20 has a cut-off threshold different with respect to the cut-off threshold of the filtering membrane 13 of the separation filter 10: in particular, the filtering membrane 23 of the concentration filter 20 has a lower cut-off threshold with respect to the membrane 13 of the separation filter.

The filtering membrane 13 is made of porous material, in particular it may be made of synthetic material, e.g. PA, PE, PES, PS, PTFE, PAN, Nylon or others, or of filtering materials of natural origin In size terms, the filtering membrane 13 of the separation filter 10 has a cut-off threshold comprised between 50 and 200 µm, in particular equal to 100 µm: this allows preventing the flow, through the separation filter 10, of particles having greater size than the size cut-off threshold and allows the passage of particles having smaller size.

The filtering membrane 23 of the concentration filter 20 is also made of porous material, in particular it may be made of synthetic material, e.g. PA, PE, PES, PS, PTFE, PAN, Nylon or others, or of filtering materials of natural origin. In size terms, the filtering membrane 23 of the concentration filter 20 has a cut-off threshold comprised between 3 and 10 µm, in particular equal to 5 µm: this allows preventing the flow, through the concentration filter 20, of particles having size greater than the respective size cut-off threshold and allows the passage of particles having size smaller than such threshold.

The concentration circuit 3 comprises at least one supply conduit 30, as shown in each of the enclosed figures, comprising a first end 31 connectable to a supply unit 5 of the biological material 2, and a second end 32 connected to the first access 11 of the separation filter 10, in order to define a fluid communication between the supply unit 5 and the separation filter 10. The supply conduit 30 may be a tube made of plastic material, e.g. a flexible tube made of silicone material, of PVC, of PP or still another material. The supply conduit 30 may comprise flow adjustment means such as an interception member 84 which may be configured at least in an open position and in a closed position, respectively to allow or prevent the flow of fluid through the supply conduit 30. The interception member 84 may also be configured to reduce the flow of the fluid passing through the supply conduit 30. The interception member 84 may be a pump, for example a peristaltic pump, a valve or a clamp, as shown in figure 1, configured to act on the external surface of the supply conduit 30 so as to deform it and close the passage, so as to define the closed position. The clamp may be manually activated by an operator in order to define the open and closed positions: in particular, the clamp has a locking system configured to stably maintain the closed position. When an operator deactivates the locking system, the clamp allows the passage from the closed position to the open position. If the interception member 84 is a valve, the latter may be manually activatable by an operator between the open and closed positions. In a further configuration, the valve of the interception member 84 is automatically actuatable by an actuator, the latter electrically activatable, and connected to a control unit 110 configured to control it to the open position or closed position. Hereinbelow, further interception members active on different sections of the device 1 will be illustrated: such further interception members may have the same characteristics mentioned above relative to the interception member 84 of the supply conduit 30. In the enclosed figures, the interception members are schematically shown with opposite, separate and empty triangles in order to represent the open position, and with opposite, approached and solid triangles in order to represent the closed position.

In a particular configuration, the concentration circuit 3 comprises the supply unit 5, the latter defined by a first tank connected in a fluid-tight manner to the first end 31 of the supply conduit 30 and configured to accommodate the biological material 2: the first tank may be a bag made of plastic material or a rigid container, preferably sealed so as to prevent the contamination of the biological material 2 contained therein. Optionally, the first tank may be openable in order to allow the filling thereof with the biological material 2.

The concentration circuit 3 has a concentration section 40 interposed between the separation filter 10 and the concentration filter 20, and connected between the second access 12 of the separation filter 10 and the first access 21 of the concentration filter 20.

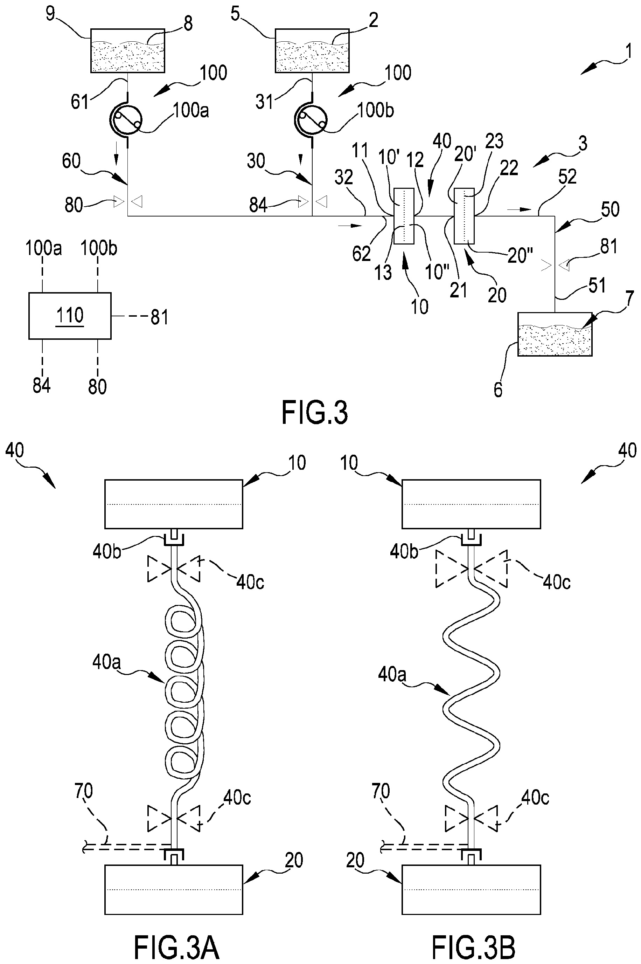

As is visible in figure 3A, the concentration section 40 may preferably be made in the shape of an elongated hollow body, e.g. a tube 40a (with rectilinear axis, curvilinear linear or spiral, the latter alternative being represented in figure 3A), so as to prevent non-uniform distributions of the treated biological material and simultaneously allow the ordered emptying in laminar operative conditions, without originating local recirculations, also allow an easy volumetric control of the extraction. In figure 3a, it is possible to see a possible embodiment of the concentration section 40 which is of tubular conformation having helically-shaped elongated shape, in particular cylindrical helical shape; figure 3B shows a variant in which the concentration section has undulated conformation. However, the concentration section may alternatively have the shape of a rectilinear elongated tube: in each case, due to the elongated tubular conformation, it ensured as already underlined above, that the material entering the section proceeds in an ordered manner, such that the material first entering into the section 40 is substantially the first to exit from the section itself (first in - first out).

The concentration section 40 defines an inner volume configured to accommodate a fraction of interest 2a of the biological material 2 and has an external casing preferably made of plastic material with the inner volume for example comprised between 10 and 100 cm³. The concentration section 40 defines a concentration volume communicating with the rest of the circuit 3 exclusively through the filtering membranes 13, 23 respectively of the separation filter 10 and of the concentration filter 20, except for the presence of a possible withdrawal conduit for collecting of the fraction of interest 2a of the biological material.

For this purpose, it must be observed that the concentration section 40 usable in the examples pursuant to figures 1 to 3 may be removable from the rest of the circuit (hence the presence of any collection channel for the fraction of interest of the biological material is not necessary). For example, as shown in fig. 3A and in fig. 3B, the concentration section may comprise, at the opposite ends thereof, connectors 40b removably engageable with corresponding counter-connectors carried by the circuit 3 (e.g. by the concentration filter and by the separation filter). Each of the connectors 40b may incorporate a check valve which closes the respective connector 40b as soon as it is disengaged from the circuit 3 or valves 40c active on the tube 40a may be provided, each operating in immediate vicinity of the respective connector 40b. Whether the concentration section is removable or not removable from the rest of the circuit 3, the concentration circuit 3 may further comprise a withdrawal conduit 70 (for collecting the fraction of interest 2a of the biological material) whose withdrawal point is situated at a point of the concentration section 40, i.e. of the tube 40a, close to the concentration filter 20.

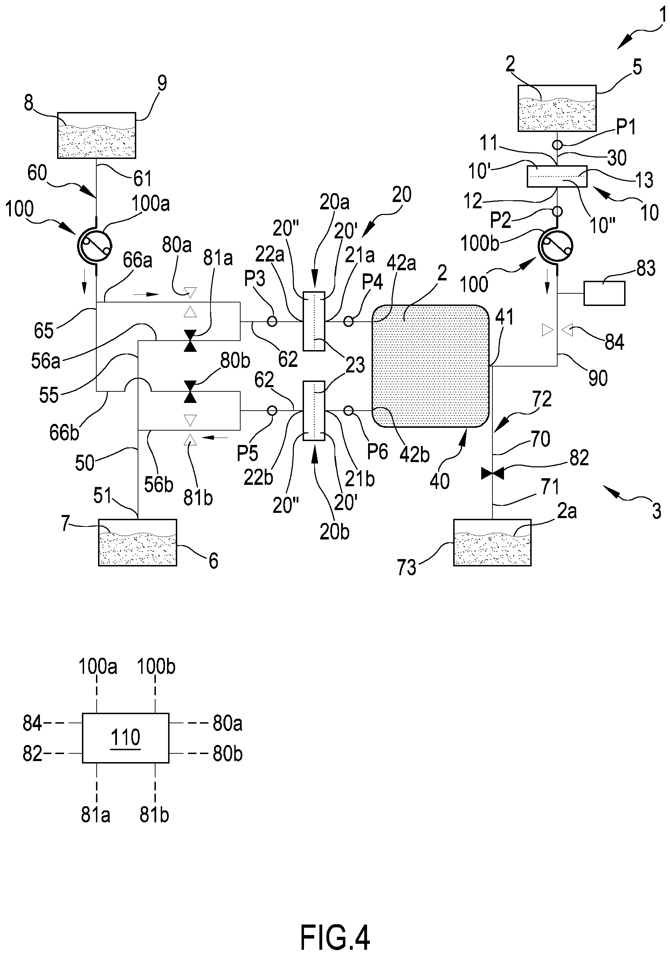

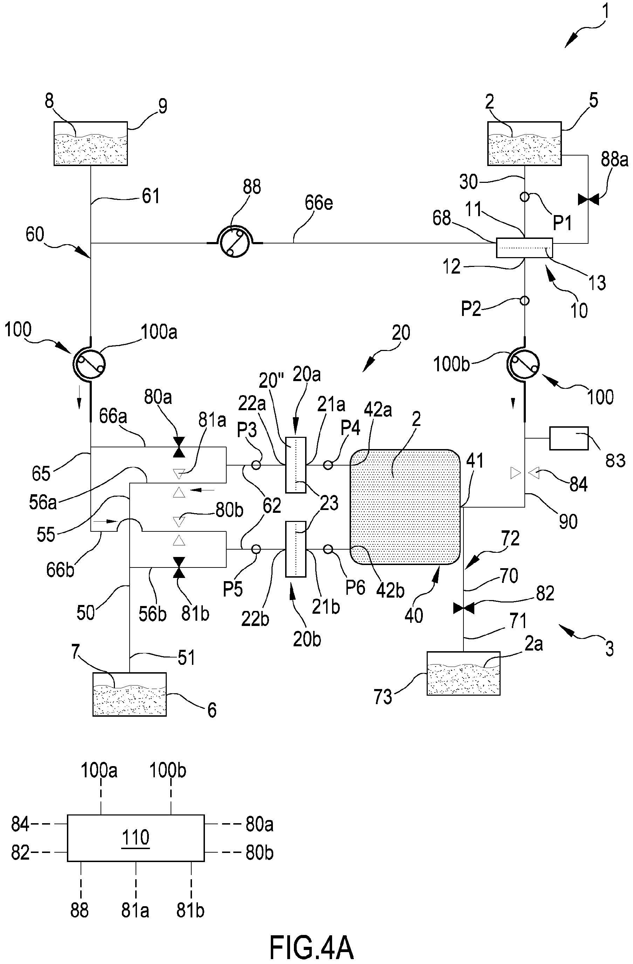

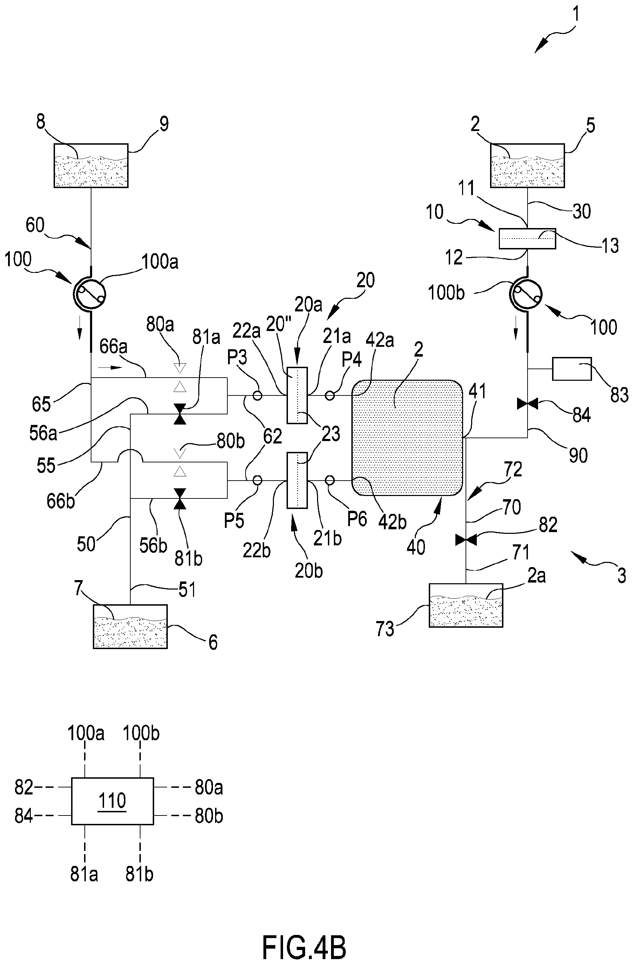

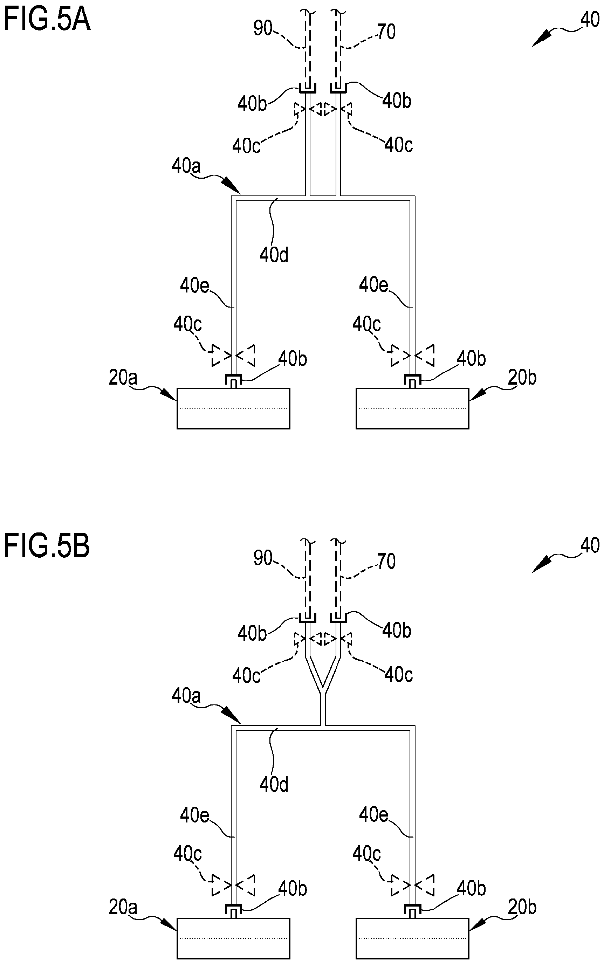

Also the concentration section 40 usable in the examples pursuant to figures 4 to 5B (see below for a more detailed description) may be removable from the rest of the circuit. For example, as shown in fig. 5A and in fig. 5B, such concentration section may comprise a tubular main body 40a having symmetrical conformation (e.g. U-shaped or V-shaped) comprising two symmetrically opposed sections 40e emerging from a base section 40d; each of the opposite sections 40e is connected with a respective of first and second concentration filters 20a, 20b, while the base section 40d, preferably in central position, is connected with the delivery channel 90 and with the withdrawal conduit 70; in figure 5A the case is shown in which the delivery channel and the withdrawal channel are connected to the base section at separate points, equidistant with respect to an ideal plane of symmetry of the section 40, while in figure 5B the delivery channel and the withdrawal channel join into a common section before being connected with the base section 40d, still with the concentration section 40 having an overall symmetry relative to a n ideal median plane perpendicular to the plane of the views of figures 5A and 5B. At the ends of the opposite sections 40e and at the connections of the base section 40d with the delivery channel 90 and with the withdrawal conduit 70, connectors 40b are provided that are removably engageable with corresponding counter-connectors carried by the circuit 3 (e.g. carried by each of the two separation filters, by the delivery conduit 90 and by the withdrawal conduit as shown in the example of figure 5). Each of the connectors 40b may incorporate a check valve that closes the respective connector 40b as soon as it is disengaged from the circuit 3 or valves 40c (see dash lines) may be provided active on the tube 40a and each operating in immediate vicinity to the respective connector 40b.

In all of the above-described examples, during the operation of the device 1, the biological material 2 is configured to flow from the supply unit 5, cross the separation filter 10 passing through membrane 13 in order to access the concentration section 40, and then leave the latter by crossing the concentration filter or filters 20, 20a, 20b passing through the membrane or membranes 23.

The device 1 also comprises at least one infusion line 60 having a first end 61 connectable to a source 9 of auxiliary fluid 8 and at least one second end 62 connected to the concentration circuit 3. The auxiliary fluid source 8 may be defined by a second tank connected to the first end 61 of the infusion line 60. The infusion line 60 is configured to infuse, in the concentration circuit 3, the auxiliary fluid 8: the latter may be a saline solution. For example, the auxiliary fluid 8 may be a saline solution of sodium chloride in purified water (0.9% W/V, i.e. 9 g of NaCl per liter of H2O) with substantially isotonic characteristics. Other solutions could be used by maintaining the correct osmolality, even introducing other components capable of nourishing the cells (e.g. glucose solutions), or of stabilizing the pH (e.g. Ringer's solution), or of attaining specific functions relative to the cell population contained in the biological material 2 and for the purposes of the processing thereof, stimulating its expansion, differentiation or acquisition of principal characteristics.

The main function of the auxiliary fluid 8 is that of diluting the matrix in which the cell population is contained, causing the reduction in the concentration of the contaminants possibly present, such as oily residues, blood residues, etcetera. As will be better illustrated hereinbelow, the auxiliary fluid 8 - in the embodiments pursuant to figures 4 to 5A - is used for washing, in counter-flow, the filtering membranes of the filters 20, 20a, 20b. In addition, the auxiliary fluid 8 may also perform the function of "piston" in the concentration section 40 (in particular when this has the shape of tube 40a - see fig. 3A) in order to push the fraction of interest of the biological material towards and out from the withdrawal conduit 70, hence facilitating of the step for extracting the cellular concentration. The infusion line 60 preferably comprises an infusion flow regulator, for example including at least one interception member 80 which may be configured at least in an open position and in a closed position, respectively to allow or prevent the flow of fluid through the infusion line 60.The interception member may be clamp or a valve or even a peristaltic pump or a combination thereof. The interception member 80 may also be configured to reduce the flow of fluid crossing the infusion line so as to control the flow rate of auxiliary fluid 8 entering the concentration circuit 3.

In the embodiment shown in figures 4, 4A, 4B, 5 and 5A, the concentration circuit 3 may for example also comprise a delivery channel 90 interposed between the second access 12 of the separation filter 10 and an intake port 41 of the concentration section 40: the delivery channel 90, connected with the second access 12 of the separation filter 10 and with the intake port 41 of the concentration section 40, is thus configured in order to place the separation filter 10 in fluid communication with the concentration section 40. The delivery channel 90 may be a tube made of rigid or flexible material, e.g. of silicone material, PVC, PP or other.

The concentration circuit 3 also comprises a discharge conduit 50 having a first end 51 connectable to a discharge unit 6, and at least one second end 52 connected to the second access 22 of the concentration filter 20 downstream of the concentration section 40, in order to define a fluid communication between the second access 22 of the concentration filter 20 and the discharge unit 6. In one embodiment, the concentration circuit 3 comprises the discharge unit 6, the latter defined by a collection tank connected to the first end 51 of the discharge conduit 50 and configured to accommodate a discard fraction 7 of the biological material 2 exiting from the concentration filter 20: the tank may be a bag made of plastic material or a rigid container. The discharge conduit 50 also comprises a discharge flow regulator, which may include at least one interception member 81 which may be configured at least in an open position and in a closed position, respectively to allow or prevent the flow of fluid through the discharge conduit 50. The interception member 81 may also be configured to reduce the flow of fluid as a function of need during the operation of the device 1, for example for controlling the flow rate of discharge fluid crossing the discharge conduit 50. The interception member 81 may be a clamp or a valve or a peristaltic pump or a combination thereof.

The concentration circuit 3 may comprise a number of flow adjustment means such as at least one pump 100, shown in figures 3, 4, 4A, 4B, 5 and 5A, active on the supply conduit 30, and/or on the infusion line 60 (and thus part of the infusion flow regulator) and/or on the delivery channel 90 (and thus part of the delivery flow regulator). In particular, at least one pump 100 may comprise a pump 100a active on the infusion line 60 and a pump 100b active on the supply conduit 30 or on the delivery channel 90. Each pump 100 (i.e. the pumps 100a and 100b) is preferably a peristaltic pump electrically activated by means of a motor and controlled by the control unit 110, the latter configured in order to determine the activation thereof, the turning off and the adjustment of the revolutions. In particular, the control unit 110 is configured to control the speed of the pump 100, 100a, 100b so as to control the flow rate of biological material 2 or of auxiliary fluid 8 through the concentration circuit 3 and the infusion line 60. More generally, the control unit 100 controls all the pumps present so as to adjust both the flow through each respective line (proportional to the angular speed), and the total volume supplied through each line (proportional to the cumulative angle of the motion), within the limits of volumetric precision of the actuators used.

The concentration circuit 3 can also comprise a withdrawal conduit 70, shown in figures 4, 4A, 4B, 5 and 5A, having a first end 71 connectable to a withdrawal unit 73, and a second end 72 connected to the concentration section 40: the withdrawal conduit 70 is configured to allow withdrawing of a fraction of interest 2a of the biological material 2 from the concentration section 40. The withdrawal conduit 70 may be a tube made of plastic material, e.g. of silicone material, PVC, PP or other. The withdrawal conduit 70 has a withdrawal flow regulator including at least one interception member 82 which may be configured at least in an open position and in a closed position, respectively to allow or prevent the flow of the fraction of interest 2a of the biological material 2 through said withdrawal conduit 70. Furthermore, in an embodiment not shown in the enclosed figures, the withdrawal conduit 70 may have a pump (also part of the withdrawal flow regulator) configured to determine the flow of the fraction of interest 2a of the biological material along the withdrawal conduit 70 and towards the withdrawal unit 73.

The device 1 may be associated with or it may comprise a control unit 110 connected to flow adjustment means, and to the flow regulators described above and in the current case active on at least one from among the pump 100a, the pump 100b and on the interception members 84, 80, 81 and 82 respectively of the supply conduit 30 or of the delivery channel 90, of the feed line 60, of the discharge conduit 50 and of the withdrawal conduit 70. The control unit 110 is then configured to independently control the activation or the stop of the pump 100a, of the pump 100b and of the interception members 84, 80, 81 and 82.

The concentration circuit 3, the infusion line 60, and optionally the withdrawal conduit 70, define a single circuit made of plastic material of the sterile or sterilizable disposable type. Optionally the latter further comprises the first tank, the second tank, the collection tank of the discharge unit 6, and the withdrawal unit 73. It should be observed that the concentration circuit 3 or at least the concentration section 40 may be part of a bioreactor, usable for subsequent cellular expansion, preventing the extraction of the cellular concentrate in order to then insert it into a separate bioreactor.

Hereinbelow, different embodiments of the treatment device 1 in accordance with the present invention are described in more detail.

### First embodiment of the device 1

A first embodiment, in accordance with the preceding description and shown in figures 1, 2 and 3, provides that the device 1 comprise a single separation filter 10 and a single concentration filter 20, connected to each other in series: the biological material 2 is then configured for crossing the supply conduit 30, crossing the separation filter 10, moving through the concentration section 40, and crossing the concentration filter 20, in a manner such that a discard fraction 7 of the biological material 2 exiting from the concentration filter 20 flows along the discharge conduit 50 up to the discharge unit 6: for greater clarity, figures 1 to 3 schematically show the direction of the flow along each of the sections of the circuit.

In accordance with the first embodiment of figures 1 and 3, the infusion line 60 has the second end 62 connected to the supply conduit 30 at a connection node, the latter located upstream of the first access 11 of the separation filter 10, so as to allow, at least in an operative condition, the mixing of the biological material 2 with the auxiliary fluid 8 upstream of the separation filter 10. This allows diluting the biological material 2 upstream of the separation filter 10. The dilution serves to purify and clarify the liquid matrix, i.e. reduce the concentration of the contaminants.

As shown in figure 2, the first embodiment can furthermore provide for a further second end 62 of the infusion line 60 connected to the concentration section 40 to allow, at least in an operative condition, an injection of auxiliary fluid 8 directly within the concentration section 40. This allows diluting the biological material 2 downstream of the separation filter 10 and upstream of the concentration filter 20, so as to limit the risk of clogging the concentration filter 20 or to reduce the frequency of the cleaning or substitution operations. The first embodiment may also present a configuration, not shown in the enclosed figures, in which the second end 62 of the infusion line 60 is only connected to the concentration section 40, without having any connection with the supply conduit 30 or with the first access 11 of the separation filter 10.

As shown in figures 1 to 3, the first embodiment of the device 1 provides for the presence of an infusion flow regulator, a supply flow regulator and a discharge flow regulator (respectively including interception members 80, 84, and 81, respectively active on the infusion line 60, on the supply conduit 30 and on the discharge conduit 50), controllable into the open or closed position manually or by means of a control unit 110. In particular the interception members 84 and 80 of the supply conduit 30 and of the infusion line 60 may be independently controlled so as to control the level of mixing of the biological material 2 with the auxiliary fluid 8. Figures 1, 2 and 3 show the device 1 in an operative condition in which the interception members 80, 84, and 81 are arranged in an open position.

As shown in figure 3, the first embodiment of the device 1 may have the infusion flow regulator including a first pump 100a, for example of peristaltic type, active on the infusion line 60 and configured to determine the flow of the auxiliary fluid 8 towards the separation filter 10. The device 1 may also have the supply flow regulator comprising a second pump 100b, for example of peristaltic type, connected to the supply conduit 30 and configured to determine the flow of the biological material 2 towards the separation filter 10. In particular the pump 100a and the pump 100b respectively of the infusion line 60 and of the supply conduit 30 contribute to determining the flow of the biological material 2 mixed with the auxiliary fluid 8 through the separation filter 10 and the concentration filter 20, and along the discharge conduit 50.

The first embodiment of the device 1, as shown in figures 1 to 3, may comprise the supply unit 5, the source 9 and the collection unit 6, to define a closed circuit, preferably sterile or sterilizable and disposable.

### Second embodiment of the device 1

A second embodiment of the device 1 is shown in figures 4, 4A and 4B in different operative conditions. The device 1 of the second embodiment, in addition to what described above, has the concentration filter formed by a first and a second concentration filter 20a, 20b, each of these placed downstream of the concentration section 40. Note that more than two concentration filters could be provided. Each first and second concentration filter 20a, 20b has the characteristics described above in relation to the concentration filter 20: in particular the first and the second concentration filter 20a, 20b each have a first access 21a, 21b and a respective second access 22a, 22b, in which the first access 21a, 21b of each first and second concentration filter 20a, 20b is connected to the concentration section 40, while the second accesses 22a, 22b are connected to the infusion line 60 and to the discharge conduit 50, with the ability to selectively create a fluid communication either with the infusion line (for example with one branch of the infusion line or with a respective independent conduit of the infusion line) or with the discharge conduit (for example with a branch of the discharge conduit or with an independent channel of the discharge conduit).

In particular the first access 21a of the first concentration filter 20a is connected to a first connection port 42a of the concentration section 40, while the first access 21b of the second concentration filter 20b is connected to a second connection port 42b of the concentration section 40: the first and the second connection ports 42a, 42b are preferably separate from each other. The first accesses 21a, 21b may be connected directly to the concentration section 40, or by means of the interposition of a connection section defined, for example, by a connection tube.

In accordance with the second embodiment, the infusion line 60 comprises a common primary section 65 terminating in the first end 61 connectable or connected to the supply unit 5. The infusion line 60 also comprises a first secondary section 66a which terminates in a second end 62 of the infusion line 60 and which connects the primary section 65 with the second access 22a of the first concentration filter 20a. In addition, the infusion line 60 comprises a second secondary section 66b which terminates in another second end 62 of the infusion line 60 and which connects the primary section 65 with the second access 22b of the second concentration filter 20b (note that alternatively the first and second secondary sections could be independent lines connected to the auxiliary fluid source or to one respective auxiliary fluid source - in other words the infusion line may include a common primary section bifurcating into two secondary sections as shown in figures 4, 4A, 4B or two parallel infusion channels). The primary section 65, the first secondary section 66a and the second secondary section 66b may be tubes of plastic material, e.g. flexible tubes made of silicone material, of PVC, of PP or still another material. As shown in figures 4, 4A, 4B, the first and the second secondary section 66a, 66b of the infusion line 60 each comprise an infusion flow regulator including a respective interception member 80a, 80b which may be configured at least in an open position and in a closed position, and having the same characteristics previously described in relation to the interception members previously introduced in the present description.

In accordance with the second embodiment, the discharge line 50 of the concentration circuit 3 in turn comprises a common primary section 55 terminating in the first end 51 connectable or connected to the discharge unit 6 by means of the discharge conduit 50. The discharge line 50 also comprises a first secondary section 56a which terminates in a second end 52 of the discharge line 50 and which connects the primary section 55 with the second access 22a of the first concentration filter 20a. In addition, the discharge line 50 comprises a second secondary section 56b which terminates in another second end 52 of the discharge line 50 and which connects the primary section 55 with the second access 22b of the second concentration filter 20b (similar to what discussed for the infusion line, also the discharge line may have a bifurcating design as described or, alternatively, may include two separate channels leading to a single discharge unit or to separate discharge units). The first and the second secondary section 56a, 56b of the discharge line 50 each comprise a discharge flow regulator including a respective interception member 81a, 81b which may be configured at least in an open position and in a closed position, and having the same characteristics described above in relation to the interception members previously introduced in the present description.

In one configuration, the second embodiment comprises the tank of the supply unit 5, the source 9 of the feed line 60, and the tank of the collection unit 6, as shown in figures 4, 4A and 4B.

The device 1 may comprise a delivery channel 90 which connects the second access 12 of the separation filter 10 to an intake port 41 of the concentration section 40. Figure 4 shows a configuration where pump 100b, configured to determine the flow of the biological material 2 towards the concentration section 40, and interception member 84, are both active on the delivery channel 90: alternatively, the pump 100b and/or the interception member 84 may be active on the supply conduit 30 (embodiment not shown in the enclosed figures). The pump 100b and the interception member 84 are part of a supply flow regulator, which obviously could include means different from those specifically described here by way of example.

In a preferred embodiment, the intake port 41 is substantially equidistant from the first and second connection port 42a, 42b: in particular, the intake port 41 is arranged preferably on a side that is opposite to the connection ports 42a, 42b. This allows ensuring a balanced flow of biological material 2 and of the auxiliary fluid 8 towards the first and the second concentration filters 20a, 20b. Furthermore, the first and the second connection ports 42a, 42b are preferably spaced from each other so as to be positioned symmetrically from each other, with respect to an ideal axis/plane intercepting the intake port 41.

The device 1 may also comprise a gas vent line 83 configured for placing the concentration circuit 3 in communication with the external environment: in particular the vent line is configured to allow the exit of possible gas or air bubbles present within the concentration circuit 3, preventing contamination of the contents of the closed circuit with contaminant particles of physical or biological nature coming from the external environment. The vent line may comprise a hydrophobic filter configured to allow the passage of gas and at the same time prevent the passage of solids or liquids through the vent line. Alternatively or additionally, the gas vent line 83 comprises a ventilation valve which may be configured between an open position, in which it allows the discharge of gas present within the device (e.g. air) through the vent line 83, and a closed position, in which it prevents the passage of gas and liquid through the vent line. Nevertheless, as indicated above, additionally or alternatively to the vent line 83, the separation filter 10 may comprise at least one vent for air present in the first and/or in the second chamber 10', 10" and provided with a respective hydrophobic membrane then capable of venting possible air present in the circuit, and the concentration filter may in turn comprise at least one vent for air present in the first and/or in the second chamber 20', 20" and provided with a respective hydrophobic membrane capable of venting possible air present in the circuit, preventing the contamination of the contents of the closed circuit with contaminant particles of physical or biological nature coming from the external environment.

The infusion line 60 may also comprise (variant of figure 4A) a section 66e having a second end 62 connected to a connection port 68 entering in the first chamber 10' of the separation filter 10 tangentially with respect to the respective filtering membrane 13, as shown in figure 4A where an additional discharge line 66f (which may be in turn connected to the container 5 or to a discharge) is visible which may be selectively open (by suitably actuating a discharge valve 88a placed on such recirculation line) and hence crossed by a flow determined for example by controlling a wash flow regulator configured (under the command of control unit 110) for delivering to the first chamber 10' a tangential wash flow; for example the wash flow regulator may include a washing pump 88 active on the section 66e. The tangential wash may be caused by action of the control unit 110 on the wash flow regulator such as to take place either continuously or at regular intervals or at intervals determined by detection of a triggering event which is either a user command received by the control unit, or expiration of set timeout period, or a detection of reaching a specific transmembrane pressure across the separation filter (10) as detected based on pressure signals from sensors (p1 and p2) positioned upstream and downstream the separation filter and communicatively connected to the control unit (110); this allows defining, at least in an operative condition, a flow of auxiliary fluid 8 that is tangential with respect to the surface of the filtering membrane 13, facilitating the removal of the residues of biological material 2 accumulated on the filtering membrane 13 and increasing the operating efficiency of the filter 10.

In a currently preferred variant pressure sensors p1 and p2 are respectively located upstream and downstream of the separation filter 10, as shown in figure 4A. The control unit 110 is configured for receiving the pressure signals from the sensors p1 and p2, determining the transmembrane pressure across the membrane 13 of the separation filter 10 (TMP=p2-p1) and, when the transmembrane pressure exceeds a predetermined threshold indicative of an excessive clogging of the filter 10, controlling the opening of the discharge valve 88A (if present) and the activation of the washing pump 88, for example by maintaining the discharge valve 88A open and the washing pump 88 active for a predetermined time interval.

In a further configuration, a second end 62 of the infusion line may be connected to a connection port entering the first chamber 20' of the first and/or second concentration filter 20a, 20b: as will be better illustrated hereinbelow, the auxiliary fluid 8 is alternatively sent to the first chamber 20' of the first concentration filter or to the first chamber 20' of the second concentration filter so as to create a sequence type for 'push-pull' of fluid through the two concentration filters 20a, 20b, facilitating the removal of residues of biological material 2 accumulated on the filtering membrane 23, in order to define a washing adapted to increase the operating efficiency also of the filters 20a and 20b. As will be seen the 'push-pull' sequence may be managed temporally or by using pressure signals.

In particular, the device 1, in accordance with the second embodiment, is configurable in a first filtering condition shown in figure 4, in a second filtering condition shown in figure 4A and in an condition of withdrawal shown in figure 4B.

In particular the first filtering condition may be defined as follows, again referring to the non-limiting examples shown:
- the interception member 80a of the first secondary section 66a of the infusion line 60 is arranged in the open position;
- the interception member 81a of the first secondary section 56a of the discharge conduit 50 is arranged in the closed position;
- the interception member 80b of the second secondary section 66b of the infusion line 60 is arranged in the closed position;
- the interception member 81b of the second secondary section 56b of the discharge conduit 50 is arranged in the open position.

The second filtering condition, alternative to the first filtering condition, is instead defined as follows:
- the interception member 80a of the first secondary section 66a of the infusion line 60 is arranged in the closed position;
- the interception member 81a of the first secondary section 56a of the discharge conduit 50 is arranged in the open position;
- the interception member 80b of the second secondary section 66b of the infusion line 60 is arranged in the open position;
- the interception member 81b of the second secondary section 56b of the discharge conduit 50 is arranged in the closed position.

In the first and the second filtering condition, the interception member 84 of the supply conduit 30 or of the delivery channel 90 is arranged in the open position to allow the flow of the biological material 2 towards the concentration section.

In other words, in the first filtering condition (figure 4), the auxiliary fluid 8 flows from the source 9 within the primary section 65 of the infusion line 60: the auxiliary fluid 8 continues along the first secondary section 66a towards the first concentration filter 20a; in particular, the auxiliary fluid 8 crosses the first concentration filter 20a via the second access 22a, through the membrane 23 and via the first access 21a and is introduced within the concentration section 40 in which mixing of the auxiliary fluid 8 with the biological material 2 takes place. Analogously, biological material 2 flows from the supply unit 5 along the supply conduit 30, at least in part crosses the separation filter 10 in a direction from the first to the second access 11, 12, crossing membrane 13 and arriving at the delivery channel 90 and then accesses the concentration section 40. The auxiliary fluid 8 and an undesired fraction of the biological material 2 present in the concentration section 40 may then cross the second concentration filter 20b from the first access 21b, through the membrane 23 of the second concentration filter towards the second access 22b. The undesired fraction of the biological material 2 exiting from the second concentration filter and defining the waste fluid 7 flows along the second secondary section 56b of the discharge conduit 50 in order to reach the collection unit 6.

Analogously, in the second filtering condition (figure 4A), the auxiliary fluid 8 flows from the source 9 within the primary section 65 of the infusion line 60: the auxiliary fluid 8 then continues along the second secondary section 66b towards the second concentration filter 20b; in particular, the auxiliary fluid 8 crosses the second concentration filter 20b flowing from the second access 22b, through the membrane 23 of the second concentration filter, to the first access 21b and is introduced within the concentration section 40 where the mixing of the auxiliary fluid 8 with the biological material 2 takes place. The auxiliary fluid 8, while crossing of the second concentration filter 20b from the second access 22b to the first access 21b, also washes the respective filtering membrane 23, totally or partially removing any accumulation of the particles of biological material 2 possibly retained by the filtering membrane 23 during the first filtering condition. The auxiliary fluid 8 and an undesired further fraction of the biological material 2 cross, in the second filtering condition, the first concentration filter 20a passing from the first access 21a towards the second access 22a of the first concentration filter. The undesired fraction of the biological material 2 exiting from the first concentration filter 20a and defining the waste fluid 7 flows along the first secondary section 56a of the discharge conduit 50 in order to reach the collection unit 6.

The device 1 is cyclically configurable in the first and in the second filtering condition, so as to cyclically wash respectively the first and second concentration filters 20a, 20b. As mentioned above, the control unit 110 is configured for switching the device 1 from the first to the second filtering condition at predetermined time intervals or following detection of a given triggering even, e.g., the attainment of specific transmembrane pressure conditions (TMP) at the first and second concentration filters. Also in this case, pressure sensors p3 and p4 may be respectively located upstream and downstream of the first concentration filter 20a and pressure sensors p5 and p6 may be respectively located upstream and downstream of the second concentration filter 20b, as shown in figure 4A. The control unit 110 is configured to control the device 1 in the first filtering condition, to receive the pressure signals from the sensors p3, p4, to then determine the transmembrane pressure across the membrane of the first concentration filter (TMP=p4-p3) and, when the transmembrane pressure across the first concentration filter exceeds a predetermined threshold indicative of an excessive clogging, to command the switch to the second filtering condition in which the infusion of the auxiliary fluid 8 takes place towards the second of the two concentration filters. The control unit 110 is also configured to control the device 1 in the second operative condition, to receive the pressure signals from the sensors p5, p6, to then determine the transmembrane pressure straddling the membrane of the second concentration filter (TMP=p6-p5) and, when the transmembrane pressure straddling the second concentration filter exceeds a predetermined threshold indicative of an excessive clogging, to control switch back to the first operative condition in which the infusion of the auxiliary fluid 8 takes place towards the first of the two concentration filters. The described procedure or concentration cycle - whether managed temporally or using the TMP or other triggering event - may be cyclically repeated by the control unit.

The treatment device 1 is also configurable in a condition of withdrawal to allow withdrawing of the fraction of interest 2a of the isolated biological material present within the concentration section 40, in particular after execution a plurality of consecutive time of the above described concentration cycle. The condition of withdrawal, shown in figure 4B, may be defined as follows:
- the interception member 84 on the supply conduit 30 or on the delivery channel 90 is in closed position;
- at least one from between the interception member 80a, 80b of the first and second secondary sections 66a, 66b of the infusion line 60 is in open position;
- the interception member 81a, 81b of the first and second secondary sections 56a, 56b of the discharge conduit 50 is in closed position;
- the interception member 82 on the withdrawal conduit 70 is in open position.

In particular, figure 4B shows a withdrawal condition in which both the interception members 80a, 80b of the first and second secondary sections 66a, 66b of the infusion line 60 are in open position. Alternatively, the interception members 80a, 80b of the first and second secondary sections 66a, 66b of the infusion line 60 are opened one at a time, in two separate steps, controlling the volume introduced in each of the two halves of the concentration section 40 so as to accurately measure the volumetric quantity of material pushed towards the discharge conduit 50. In other words, in the withdrawal condition, the auxiliary fluid 8 flows from the source 9 along the primary section 65 of the infusion line 60, crosses the first and/or the second secondary section 66a, 66b, accesses the concentration section 40 in order to push the fraction of interest 2a of the biological material 2, so as to drive the fraction of interest 2a towards the end of the concentration section 40 where the withdrawal point of the discharge conduit 50 is provided, then making the fraction of interest of the biological material flow along the withdrawal conduit 70 and towards the collection tank 73.

The second embodiment of the device 1 also comprises the/a control unit 110 connected to each interception member 80a, 80b of the first and second secondary sections 66a, 66b of the infusion line 60, to each interception member 81a, 81b of the first and second secondary sections 56a, 56b of the discharge conduit 50, and to the interception member 84 of the delivery channel 90 or of the supply conduit 30. The control unit 110 is configured to independently control each of the interception members 81a, 81b, 80a, 80b and 84 to be arranged in a respective open or closed position. Furthermore, the control unit is connected to the pump 110a and to the pump 110b respectively of the infusion line 60 and of the delivery channel 90 and configured to control the activation, stop and manage the rotation speed thereof.

The control unit 110 is configured to perform a first procedure, defining the first filtering condition, comprising:
- activating the second pump 100b to move biological material 2 towards the concentration section 40 through the separation filter 10;
- controlling to the open position the interception member 80a of the first secondary section 66a of the infusion line 60;
- controlling to the closed position the interception member 81a of the first secondary section 56a of the discharge conduit 50;
- controlling to the closed position the interception member 80b of the second secondary section 66b of the infusion line 60;
- controlling to the open position the interception member 81b of the second secondary section 56b of the discharge conduit 50;
- activating the first pump 100a to move auxiliary fluid 8 towards the concentration section 40 through the first concentration filter 20a.

Furthermore the control unit 110 is configured to perform a second procedure, defining the second filtering condition, comprising:
- activating the second pump 100b to move biological material 2 towards the concentration section 40 through the separation filter 10;
- controlling to the closed position the interception member 80a of the first secondary section 66a of the infusion line 60;
- controlling to the open position the interception member 81a of the first secondary section 56a of the discharge conduit 50;
- controlling to the open position the interception member 80b of the second secondary section 66b of the infusion line 60;
- controlling to the closed position the interception member 81b of the second secondary section 56b of the discharge conduit 50;
- activating the first pump 100a to move auxiliary fluid 8 towards the concentration section 40 through the second concentration filter 20b.

The control unit 110 is then configured to sequentially and cyclically repeat the first and the second procedure, so as to define a continuous filtering of the biological material 2 through the separation filter 10 and the first and second concentration filter 20a, 20b, and simultaneously cyclically allowing the washing of the concentration filters 20a, 20b through the reversal of the flow direction. In other words, during the first filtering condition the device 1 allows the washing of the first concentration filter 20a and, simultaneously, the filtering of the biological material 2 through the second concentration filter 20b. Analogously, during the second filtering condition the device 1 allows the washing of the second concentration filter 20b and, simultaneously, the filtering of the biological material 2 through the first concentration filter 20a.

The control unit 110 may command switch between the first and the second operative condition as a function of a predetermined time interval, so as to periodically determine the washing of the first and second concentration filters 20a, 20b, or as a function of a triggering event (e.g., as a function of the transmembrane pressure across each one of the filters, as described above).

In an optional configuration of the second embodiment, the concentration section 40 comprises a pressure sensor configured for emitting a signal representative of the pressure inside the concentration section 40, in which the pressure sensor is connected to the control unit 110. The latter is then configured for receiving the pressure signal and controlling the device 1 to switch between the first and the second filtering condition as a function of the pressure signal representative of the pressure inside the concentration section 40. In particular the control unit 110 is configured for passing from the first to the second operative condition, and vice versa, if the pressure signal exceeds a predefined threshold. In other words, during the operation of the device 1, the residues of biological material 2 retained by the first and/or by the second concentration filter 20a, 20b can slow or prevent the flow of fluid therethrough, consequently generating an increase of pressure within the concentration section 40: the signal generated by the pressure sensor therefore allows estimating the level of blockage of the first and/or of the second concentration filter 20a, 20b, and, on the basis of this estimate, cause switching to the first or the second filtering condition.

Alternatively to that described above, it may be provided to use a transmembrane sensor system (for example comprising two separate pressure sensors or a differential pressure sensor) capable of determining the differential pressure across each of the membranes 23. The transmembrane sensor system is connected to the control unit and generates a signal that allows the control unit to estimate the level of blockage of the first and/or of the second concentration filter 20a, 20b, and, on the basis of this estimate, cause the switch between the first and the second operative condition.

The device 1 may also comprise, alternatively to in combination with the pressure sensor described above, a weight gauge, connected to the control unit 110, configured for emitting a level signal representative of the quantity of biological material 2 present in the tank 5: the weight gauge may be a scale configured to weigh the tank of the supply unit 5. The control unit 110 may be configured for receiving in input the level signal, and preferably the variation of the level signal representative of the variation of the quantity of biological material 2 present in the tank 5 during the operation of the device 1. As a function of the level signal or of its variation over time, the control unit 110 may be configured to control and specifically to switch the device 1 in the first or in the second filtering condition.

In addition, the control unit 110 is connected to the interception member 82 of the withdrawal conduit 70 and is configured to control the interception member 82 to the open or closed position. The control unit 110 may in particular be configured to define the withdrawal condition by implementing the following steps:
- stopping the second pump 100b;
- controlling to the closed position the interception member 84 arranged on the supply conduit 30 or on the delivery channel 90;
- controlling to the open position at least one from between the interception member 80a, 80b of the first and second secondary sections 66a, 66b of the infusion line 60;
- controlling to the closed position the interception member 81a, 81b of the first and second secondary sections 56a, 56b of the discharge conduit 50;
- activating the first pump 100a to determine the flow of the auxiliary fluid 8 entering the concentration section 40;
- controlling to the open position the interception member 82 of the withdrawal conduit 70.

### Process for the treatment of biological material

The present invention also concerns a process for the treatment of biological material 2: the process can use the device 1 described above in accordance with the first or the second embodiment.

Before proceeding with the actual steps for treating the biological material to be concentrated, the entire circuit 3 and in any case all the conduits of the device 1 are filled with liquid (priming procedure). For this purpose, the auxiliary solution may be used, which is made to suitably circulate in the various lines of the device so as to totally remove air or undesired particles possibly present. For example, it is possible to remove air by means of the paths for accessing and exiting from the circuit (inlet channel, discharge channel) and/or by means of specific membrane devices present on the filters 20 (vents with hydrophobic membrane) or through suitable vent lines (line 83). The priming procedure is governed by the control unit 110 with specific steps for filling, circulation of the priming liquid, discharge of the used priming liquid until obtaining a complete removal of air.

After priming the process comprises at least the following steps:
- determining a first filtering of the biological material 2 through the separation filter 10;
- determining a second filtering of the biological material 2, subsequent to the first filtering, through the concentration filter 20: the steps of first and second filtering determine concentration of a fraction of interest 2a of the biological material 2 at the concentration section 40;
- infusing the auxiliary fluid 8 in the concentration circuit through the infusion line 60 to determine a dilution of the matrix of the starting biological material 2 with the auxiliary fluid 8.

In particular, the step of infusing the auxiliary fluid 8 and the steps of determining the first and second filtering may comprise a respective sub-step of activating the first and the second pump 100a, 100b.

The concentration process, when the device has the first and the second concentration filter 20a, 20b (see second embodiment), comprises the following steps for defining the first filtering condition (see figure 4):
- activating the second pump 100b to determine the flow of the biological material 2 entering the concentration section 40 through the separation filter 10;
- controlling to the open position the interception member 80a of the first secondary section 66a of the infusion line 60;
- controlling to the closed position the interception member 81a of the first secondary section 56a of the discharge conduit 50;
- controlling to the closed position the interception member 80b of the second secondary section 66b of the infusion line 60;
- controlling to the open position the interception member 81b of the second secondary section 56b of the discharge conduit 50;
- activating the first pump 100a to determine the step for infusing the auxiliary fluid 8 in the concentration section 40 of the concentration circuit 3 through the first concentration filter 20a, along a direction directed from the second access 22a to the first access 21a of the first concentration filter 20a.

The concentration process, when the device has the first and the second concentration filter 20a, 20b (see second embodiment), also comprises the following steps for defining the second filtering condition (4A):
- activating the second pump 100b to determine the flow of the biological material 2 entering the concentration section 40 through the separation filter 10;
- controlling to the closed position the interception member 80a of the first secondary section 66a of the infusion line 60;
- controlling to the open position the interception member 81a of the first secondary section 56a of the discharge conduit 50;
- controlling to the open position the interception member 80b of the second secondary section 66b of the infusion line 60;
- controlling to the closed position the interception member 81b of the second secondary section 56b of the discharge conduit 50;
- activating the first pump 100a to determine the step for infusing the auxiliary fluid 8 in the concentration section 40 of the concentration circuit 3 through the second concentration filter 20b along a direction directed from the second access 22b to the first access 21b.

The treatment process provides for cyclically repeating the first and the second filtering condition as described in the section concerning the second embodiment.

The process can also comprise, in accordance with the first and the second embodiment, a step of withdrawing the fraction of interest 2a of the biological material 2 from the concentration section 40: the withdrawal step comprises a sub-step for controlling to open position the interception member 82 on the withdrawal conduit 70, to allow the flow of the fraction of interest of the biological material 2 along the withdrawal conduit 70 entering the withdrawal unit 73 (see figure 4B).

In accordance with the first embodiment, the withdrawal step comprises the steps of:
- stopping the second pump 100b;
- controlling to the closed position the interception member 84 of the supply conduit 30;
- activating the first pump 100a to determine the flow of the auxiliary fluid 8 entering the concentration section 40;
- controlling to the open position the interception member 82 of the withdrawal conduit 70.
- in accordance with the second embodiment, the withdrawal step comprises the steps of:
- stopping the second pump 100b;
- controlling to the closed position the interception member 84 of the supply conduit 30 or of the delivery channel 90;
- controlling to the open position at least one from between the interception member 80a, 80b of the first and second secondary sections 66a, 66b of the infusion line 60;
- controlling to the closed position the interception member 81a, 81b of the first and second secondary sections 56a, 56b of the discharge conduit 50;
- activating the first pump 100a to determine the flow of the auxiliary fluid 8 entering the concentration section 40;
- controlling to the open position the interception member 82 of the withdrawal conduit 70.

Finally, it must be observed that the concentrated fraction of interest 2a of biological material 2 may be withdrawn from the collection channel 70, for example under the control of the unit 110 as described above, or by removing the entire concentration section 40 from the rest of the circuit if, still as described above, the concentration section is removably connected to the device 1 and provided with suitable means arranged to prevent the exit of the collected material.

## Claims

1. Device (1) for treatment of biological material (2) comprising:
- a concentration circuit (3) having:
at least one supply conduit (30) comprising a first end (31) connectable to a supply unit (5) of biological material (2);
at least one concentration filter (20, 20a, 20b) in fluid communication with the supply conduit (30);
a concentration section (40) in fluid communication with the supply conduit (30) upstream of the at least one concentration filter (20, 20a, 20b), said concentration section (40) being also connected to at least one first access of the concentration filter (20, 20a, 20b) and defining an inner volume configured to accommodate a fraction of interest of the biological material (2);
at least one discharge conduit (50) comprising a first end (51) connectable to a discharge unit (6), and at least one second end (52) connected to at least one second access of the at least one concentration filter (20, 20a, 20b);
- at least one infusion line (60) comprising a first end (61) connectable to an auxiliary fluid source (8) and at least one second end (62) connected to the concentration circuit (3);
**characterized in that** said concentration filter (20) comprises at least a first concentration filter (20a) and a second concentration filter (20b);
wherein the first concentration filter (20a) has:
at least one respective first access (21a) connected to the concentration section (40),
at least one respective second access (22a),
a respective filtering membrane (23) interposed between the first access (21a) and the second access (22a) of the first concentration filter (20a);
wherein the at least one infusion line (60) is configured to be placed in fluid communication with at least one second access (22a) of the first concentration filter (20a) such that auxiliary fluid directed by the at least one infusion line into the first concentration filter (20a) via the at least one second access (22a) crosses the filtering membrane (23) of the first concentration filter (20a) to reach the concentration section (40);
wherein the at least one discharge conduit (50) is configured to be placed into fluid communication with at least one second access (22a) of the first concentration filter (20a) for receiving an undesired fraction of the biological material leaving the concentration section (40) and crossing the filtering membrane (23) of the first concentration filter (20a); and
wherein the second concentration filter (20b) has:
at least one respective first access (21b) connected to the concentration section (40),
at least one respective second access (22b),
a respective filtering membrane (23) interposed between the first access (21b) and the second access (22b) of the second concentration filter (20b);
wherein the at least one infusion line (60) is configured to be placed in fluid communication with at least one second access (22b) of the second concentration filter (20b) such that auxiliary fluid directed into the second concentration filter (20b) via the at least one second access (22b) crosses the filtering membrane (23) of the second concentration filter (20b) to reach the concentration section (40),
wherein the at least one discharge conduit (50) is configured to be placed into fluid communication with at least one second access (22a) of the second concentration filter (20b) for receiving an undesired fraction of the biological material leaving the concentration section (40) and crossing the filtering membrane of the second concentration filter (20b).

2. Device according to claim 1, wherein the concentration circuit (3) also comprises at least one separation filter (10) comprising a respective first and a respective second access (11, 12), and a filtering membrane (13) interposed between said first and second access (11, 12); wherein a second end (32) of the at least one supply conduit (30) is connected to the first access (11) of the at least one separation filter (10) and said concentration section (40) is interposed between the at least one separation filter (10) and the first and second concentration filters (20a, 20b); further wherein the filtering membrane (23) of the first and second concentration filters (20a, 20b) has a cut-off threshold different from a cut-off threshold of the filtering membrane (13) of the separation filter (10).

3. Device of claim 2, wherein the concentration section (40) is on one side connected or connectable to the second access (12) of the at least one separation filter (10) and on the other side connected or connectable to the first access (21a, 21b) of the first and second concentration filters (20a, 20b),
and/or
wherein the concentration circuit has a delivery channel (90) which connects the second access (12) of the at least one separation filter (10) to an intake port (41) of the concentration section (40), each of the first accesses (21a, 21b) of the first and second concentration filters (20a, 20b) being connected respectively to a first and a second connection port (42a, 42b) of the concentration section (40), said intake port (41) being optionally equidistant from said first and second connection ports (42a, 42b).

4. Device of claim 2 or 3, wherein the filtering membrane (23) of the first and second concentration filters (20a, 20b) has lower cut-off threshold with respect to the cut-off threshold of the filtering membrane (13) of the separation filter (10);
optionally wherein the filtering membrane (13) of the separation filter (10) has cut-off threshold of between 50 and 200 µm, and the filtering membrane (23; 23a, 23b) of the first and second concentration filters (20; 20a, 20b) has cut-off threshold of between 3 and 10 µm.

5. Device according to any one of the preceding claims comprising:
at least one infusion flow regulator (80a, 80b) operative on the at least one infusion line (60) and configured to control flow of auxiliary fluid through the same at least one infusion line,
at least one discharge flow regulator (81a, 81b) operative on the discharge conduit (50) and configured to control flow of undesired fraction of biological material through the same discharge conduit; and
a control unit (110) connected to the infusion flow regulator (80a, 80b) and to the discharge flow regulator (81a, 81b) and configured to execute the following concentration cycle:
control the infusion flow regulator (80a, 80b) to infuse auxiliary fluid (8) in the concentration section (40) alternatively through the first or second concentration filter (20a, 20b), and
control the discharge flow regulator (81a, 81b) to move undesired fractions of biological material (2) out of the concentration section (40) alternatively through the first or second concentration filter (20a, 20b), the control unit (110) being configured to control the infusion flow regulator (80a, 80b) and the discharge flow regulator (81a, 81b) so that when one of the first and the second concentration filter (20a, 20b) is crossed by the auxiliary fluid (8) entering the concentration section (40), the other between the first and second filter (20a, 20b) is crossed by an undesired fraction of the biological material leaving the concentration section (40);
in particular wherein the control unit is configured for repeating the above concentration cycle a plurality of times.

6. Device according to any one of the preceding claims, wherein the at least one infusion line (60) comprises:
a common primary section (65) terminating in said first end (61) of the infusion line (60);
a first secondary section (66a) which ends at a second end (62) of the infusion line (60) and which connects said primary section (65) with the second access (22a) of the first concentration filter (20a);
a second secondary section (66b) which ends in another second end (62) of the infusion line (60) and which connects said primary section (65) with the second access (22b) of the second concentration filter (20b);
and wherein the discharge conduit (50) of the concentration circuit (3) comprises:
a common primary section (55) terminating in said first end (51) of the discharge conduit (50);
a first secondary section (56a) which ends at one second end (52) of the discharge conduit (50) and which connects said primary section (55) with the second access (22a) of the first concentration filter (20a);
a second secondary section (56b) which ends in another second end (52) of the discharge conduit (50) and which connects said primary section (55) with the second access (22b) of the second concentration filter (20b).

7. Device according to claims 5 and 6, wherein each of said first and second secondary sections (66a, 66b) of the infusion line (60) comprises at least one respective interception member (80a, 80b) which is part of the infusion flow regulator and which is configurable at least in an open position and in a closed position, respectively to allow or prevent flow of the auxiliary fluid (8) through the first and second secondary sections (66a, 66b) of the infusion line (60) respectively into the first and second concentration filter; and/or
wherein each first and second secondary section (56a, 56b) of the discharge conduit (50) comprises at least one respective interception member (81a, 81b) which is part of the discharge flow regulator and which is configurable at least in an open position and in a closed position, respectively to allow and prevent flow of the waste fluid (7) out of the first and second concentration filters (20a, 20b) through said first and second secondary section (56a, 56b) of the discharge conduit (50).

8. Device according to any one of claims from 5 to 7, wherein the control unit (110) during execution of said concentration cycle is configured to arrange the circuit (3) alternatively in:
a first filtering condition in which the control unit controls the infusion flow regulator active on the infusion line to inject into the concentration section (40) a predetermined volumetric quantity of auxiliary fluid (8) through the first secondary filter (20a), and control said discharge flow regulator active on the discharge conduit (50) to determine the exit from the discharge conduit (50) of an equivalent volumetric quantity of biological material to be discarded through the second secondary filter (20b);
a second filtering condition in which the control unit controls the infusion flow regulator active on the infusion line to inject into the concentration section (40) a predetermined volumetric quantity of auxiliary fluid (8) through the second secondary filter (20b), and control said discharge flow regulator active on the discharge conduit (50) to determine the exit from the discharge conduit (50) of an equivalent volumetric quantity of biological material to be discarded through the first secondary filter (20a).

9. Device according to claims 7 and 8, wherein the control unit is configured to:
command the device in the first filtering condition in which:
the interception member (80a) of the first secondary section (66a) of the infusion line (60) is arranged in the open position;
the interception member (81a) of the first secondary section (56a) of the discharge conduit (50) is arranged in the closed position;
the interception member (80b) of the second secondary section (66b) of the infusion line (60) is arranged in the closed position;
the interception member (81b) of the second secondary section (56b) of the discharge conduit (50) is arranged in the open position;
command the device in the second filtering condition, alternative to the first filtering condition, in which:
the interception member (80a) of the first secondary section (66a) of the infusion line (60) is arranged in the closed position;
the interception member (81a) of the first secondary section (56a) of the discharge conduit (50) is arranged in the open position;
the interception member (80b) of the second secondary section (66b) of the infusion line (60) is arranged in the open position;
the interception member (81b) of the second secondary section (56b) of the discharge conduit (50) is arranged in the closed position;
wherein the device (1) is iteratively configurable in said first and second filtering conditions under the control of said control unit.

10. Device according to any one of claims from 2 to 9, wherein the inner volume of the concentration section (40) is of between 10 and 100 cm³ and communicates with the rest of the concentration circuit (3) exclusively through the membranes (13, 23) of the at least one separation filer (10) and of the first and second concentration filters (20a, 20b); optionally wherein the concentration circuit is a single plastic disposable component, more optionally a single plastic sterile disposable component.

11. Device according to any one of the preceding claims, wherein the at least one infusion line (60) is also configured to be selectively placed in fluid communication with a first chamber (10') of the separation filter (10) to define, at least in one operative condition, a tangential flow with respect to the surface of the filtering membrane (13), the separation filter further comprising a second chamber (10") separated from the first chamber (10') by the filtering membrane (13) of the same separation filter (10);
wherein the infusion line (60) has a section (66e) having a second end (62) connected to the first chamber (10') of the separation filter (10) and wherein an additional discharge line (66f) is also connected to the first chamber (10') of the separation filter, the or a control unit (110) being active on a wash flow regulator, optionally including a washing pump (88), active on the section (66e) for causing a tangential wash through the first chamber (10') of the separation filter and out of the additional discharge line (66f), the control unit operating the wash flow regulator either continuously or at regular intervals or at intervals determined by detection of a triggering event which is either a user command received by the control unit, or expiration of set timeout period, or a detection of reaching a specific transmembrane pressure across the separation filter (10) as detected based on pressure signals from sensors (p1 and p2) positioned upstream and downstream the separation filter and communicatively connected to the control unit (110).

12. Device according to any one of the preceding claims from 5 to 11, further comprising a withdrawal conduit (70) connected with the concentration circuit (3) and configured for collecting the fraction of interest of the biological material, and a withdrawal flow regulator (82) operative on the withdrawal conduit and configured to control flow of the fraction of interest (2a) of the biological material (2) through said withdrawal conduit (70), optionally the withdrawal flow regulator comprising at least one respective interception member (82) which is configurable at least in an open position and in a closed position, respectively to allow or prevent flow of the fraction of interest (2a) of the biological material (2) through said withdrawal conduit (70),
and wherein the control unit is connected to the withdrawal flow regulator (82) and is further configured to:
first command execution of said concentration cycle a plurality of times,
then stop execution of the concentration cycle and command the withdrawal flow regulator (2) to extract at least part of the fraction of interest (2a) of the biological material (2) from the concentration section (40) through said withdrawal conduit (70);
further wherein the withdrawal conduit (70) has a first end (71) connectable to a withdrawal unit (73), and the second end (72) connected to the concentration section (40),
optionally wherein the concentration section is removable from the rest of the circuit (3) and comprises connectors (40b) removably engageable to corresponding counter-connectors carried by the first and second concentration filters (20a, 20b) and by the separation filter (10), and wherein each of the connectors (40b) incorporates a check valve which closes the respective connector (40b) as soon as it is disengaged from the circuit (3) or wherein valves (40c) are active on the concentration section (40) and operating each adjacent to the respective connector (40b);
more optionally wherein the concentration section (40) comprises a tubular main body (40a) having a symmetrical conformation, optionally U-shaped or V-shaped, comprising two symmetrically opposed sections (40e) emerging from a base section (40d); and wherein each of the opposite sections (40e) is connected to a respective one of said first and second concentration filters (20a, 20b), while the base section 40d, preferably located in a central position, is connected to the withdrawal conduit (70) and with the selection filter (10).

13. Device according to any one of the preceding claims comprising a supply flow regulator, in particular operative on the supply conduit (30) or the delivery channel (90), configured to control flow of biological material to the concentration section (40),
wherein the control unit (110) is also configured to put the device in a withdrawal condition in which the control unit controls the infusion flow regulator to deliver auxiliary fluid into the concentration section (40) through one of said first and second filters (20a, 20b), controls the supply flow regulator to stop flow of biological material through the delivery channel or supply conduit, controls the withdrawal flow regulator to collect the concentrated fraction of interest through the withdrawal conduit (70),
optionally wherein:
the supply flow regulator comprises an interception member (84) which may be configured at least in an open position and in a closed position respectively to allow or prevent flow of biological material flow to the concentration section (40), and
the control unit in the withdrawal condition configures the device such that:
the interception member (84) on the conduit (30) or on the delivery channel (90) is in the closed position;
at least one of the interception members (80a, 80b) of the first and second secondary sections (66a, 66b) of the infusion line (60) is in the open position;
the interception member (81a, 81b) of the first and second secondary sections (56a, 56b) of the discharge conduit (50) is in the closed position;
the interception member (82) on the withdrawal conduit (70) is in the open position.

14. Device according to any one of claims 8 to 13, wherein the control unit is configured to switch the device (1) from the first to the second filtering condition either at predetermined time intervals or upon detection of a transmembrane pressure threshold across the membrane of the second concentration filter, and to switch the device (1) from the second to the first filtering condition either at predetermined time intervals or upon detection of a transmembrane pressure threshold across the membrane of the first concentration filter;
in particular wherein first pressure sensors (p3 and p4) are provided, respectively located upstream and downstream of the first concentration filter (20a) and second pressure sensors (p5 and p6) respectively located upstream and downstream of the second concentration filter (20b), the control unit 110 being configured for cyclically repeating the following steps:
a. controlling the device (1) in the first filtering condition,
b. receiving pressure signals from the second pressure sensors (p5, p6),
c. then determining the transmembrane pressure across the membrane of the second concentration filter,
d. when the transmembrane pressure across the second concentration filter membrane exceeds a predetermined threshold indicative of an excessive clogging, commanding switch of the device to the second filtering condition,
e. controlling the device (1) in the second filtering condition,
f. receiving the pressure signals from the first pressure sensors (p3, p4),
g. then determining the transmembrane pressure across the membrane of the first concentration filter,
h. when the transmembrane pressure across the first concentration filter membrane exceeds a predetermined threshold indicative of an excessive clogging, commanding switch of the device to the first filtering condition.

15. Process for the treatment of biological material (2) using the treatment device according to any one of the preceding claims, said process comprising at least the following steps:
carrying out a priming procedure for washing and removing air from the circuit (3);
feeding to the concentration section (40) biological material (2) which has undergone at least a first filtration stage; optionally wherein the first filtration stage of the biological material (2) takes place through at least one separation filter (10) forming part of the circuit (3) and placed upstream of the concentration filter (20);
infusing the auxiliary fluid (8) into the concentration section (40), alternatively through the first or second concentration filter (20a, 20b), wherein when one of the first or the second concentration filter (20a, 20b) is crossed by the auxiliary fluid (8) entering the concentration section (40), the other between the first and second filter (20a, 20b) is crossed by an undesired fraction of the biological material leaving the concentration section (40); optionally repeating the steps of feeding and infusing a number of times;
withdrawing a fraction of interest (2a) of the biological material (2) from the concentration section (40), said step of withdrawing the fraction of interest comprising: in a first alternative, at least one sub-step of causing the flow of the fraction of interest (2a) of the biological material (2) along the withdrawal conduit (70) towards a withdrawal unit (73); or, in a second alternative, separation of the concentration section (40) from the rest of the circuit (3); wherein causing the flow of the fraction of interest along the withdrawal line according to said first alternative includes the following sub-steps:
- preventing flow of fluid through the supply line,
- causing flow of auxiliary fluid through at least one of the first and second concentration filters to push the fraction of interest out of the concentration section through the withdrawal conduit, optionally wherein the process comprises controlling the volume of auxiliary fluid introduced into the concentration section (40) during said step of withdrawing the fraction of interest so as to accurately measure the volumetric quantity of material pushed towards the discharge conduit (50).
